# EUROPEAN PATENT APPLICATION

(11) **EP 4 365 303 A1**
(43) Date of publication of application: **08.05.2024**
(21) Application number: 22383053.0
(22) Date of filing: 02.11.2022
(51) Int. Cl.: C12Q 1/6806, C12N 15/10

(54) **PURIFICATION OF LINEAR DNA PRODUCTS**

(71) Applicant: 4basebio, S.L.U., 28049 Cantoblanco (Madrid) (ES); 4basebio UK Ltd, Cambridge CB24 5QE (GB)
(72) Inventor: LANCKRIET, Heikki, Cambridge, CB24 5QE (GB); PICHER, Ángel, E-28049 Cantoblanco, Madrid (ES); WALKER, Amy, Cambridge, CB24 5QE (GB)
(74) Representative: Boult Wade Tennant LLP

(57) **Abstract**

The present invention relates to the purification of linear DNA products, which may have nuclease-resistance. The linear DNA products may be double-stranded or single stranded. In addition, the present invention relates to purified linear DNA products, and their uses.

## Description

### Technical field

The present invention relates to the purification of linear DNA products, which may have nuclease resistance. The linear DNA products may be double-stranded or single stranded. In addition, the present invention relates to purified linear DNA products, and their uses.

### Background

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines. Considerable efforts have been made to extend the effective molecular lifetime of nucleic acids by increasing resistance of the nucleic acid molecules to both extracellular and intracellular nucleases. Resistance to nuclease digestion can also be accomplished by using closed DNA molecules, such as plasmids or minicircles. However, plasmids and minicircles have limited utility *in vivo* due to their frequent contamination with toxic agents derived from cell components; contamination with or bacterial or plasmid backbone sequences, such as antibiotic resistance genes; fidelity issues due to recombination events, a particular issue with polyA tails and inverted terminal repeats, that can alter the sequence of interest; and presence of different species (supercoiled, linear and open circular).

For linear molecules, one of the proposed solutions includes the use of phosphorothioated nucleotides (i.e. 2'-deoxynucleotides-5'-(α-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Alternatively, resistance to nuclease digestion may be accomplished by producing closed linear DNA molecules. For example, WO2010/086626 A1 describes a method for producing a closed linear DNA by utilizing a protelomerase. However, this method is limited in that the action of protelomerase produces the same sequence at both ends of the closed linear DNA molecule.

Further methods of producing linear DNA products have been described, such as in PCT/EP2022/061630 and PCT/EP2022/071413. These applications describe the limitations with other methods of protecting DNA against degradation, and themselves describe an important innovation in producing nuclease resistant DNA products.

Inevitably, during the manufacture of such synthetic nucleic acid molecules, there will also be produced products that have not been correctly synthesised, such as truncated molecules, those that have not annealed correctly or those that have mismatched bases, (in the case of complementary strands forming a double stranded linear portions of such DNA products). Nucleases, such as endonucleases and for example, exonuclease I, exonuclease III, exonuclease VII or exonuclease VIII or may be used to digest DNA strands that have not properly hybridised, or to digest unpaired and unhybridised strands of a duplex. However, in the case of DNA products that are protected from nuclease digestion (nuclease-resistant), some unwanted products may survive exonuclease digestion. Furthermore, proteins used in the manufacture of synthetic DNA (such as ligases and polymerases,) as well as mononucleotides and oligonucleotides, need to be removed once the linear DNA product has been synthesised.

Given the nature of the DNA molecules described in the above-mentioned PCT applications and their uses, stringent yet simple purification techniques are required in order to preserve the integrity of the DNA molecules, and ensure they are clear of impurities in order that they can be used successfully and safely in therapeutic and research settings. One of the advantages of the linear DNA products described in PCT applications PCT/EP2022/061630 and PCT/EP2022/071413 is the quick one-step process for their synthesis. As such, an equally as quick and simple purification method would be an advantage, in order to maintain the short time frame in which such linear DNA products may be produced for research or therapeutic uses. Therefore, a method of purification of linear DNA products in a simple and effective manner is still needed.

### Description of the invention

The invention provides a method for purification of a linear DNA product. By purification it is meant the separation of an intended or desired linear DNA product from unwanted DNA and other molecules (e.g., truncated products, mismatched molecules, or template DNA), unused mononucleotides and adaptor molecules, and enzymes that may be present in the reaction mixture after the synthesis of the nucleic acid product. The method of the invention is based on the presence of a binding moiety within or attached to an adaptor molecule is appended to a linear DNA molecule to form a linear DNA product to be purified. The method of the invention relies on the affinity of such a binding moiety for a substrate, with which the DNA product is contacted, such that any unbound material can be washed away. Therefore, the method for purification of a linear DNA product comprises the steps:
(a) contacting a linear DNA molecule with a ligase and a first adaptor molecule, to form a linear DNA product precursor comprising a linear region and a first adaptor molecule appended to a first end of the linear region, wherein the first adaptor molecule comprises a first binding moiety;
(b) contacting the linear DNA product with a first substrate under conditions in which the first binding moiety binds to the first substrate;
(c) washing the first substrate to remove unbound linear DNA molecules; and
(d) obtaining a purified linear DNA product.

The inventors of the present invention have discovered a method which requires a very few steps to produce and purify the linear DNA product described herein. The methods described herein are very time efficient. This is, in part, due to the fact that one adaptor molecule can perform the function of protecting the linear DNA product from nuclease activity and also assist in purification.

By binding moiety it is meant a molecule or a fragment of a molecule that is capable of binding to another molecule or molecules (a binding partner) under certain conditions. Conditions vary depending on the particular binding moiety, and will be known to the skilled person, e.g., it is known that a relatively high salt concentration encourages complementary nucleic acid strands to hybridise, in the instance of a binding moiety being a homopolymeric sequence, and its binding partner being a complementary sequence. The binding is preferably reversible. The binding may be reversed (disrupted) by changing the salt concentration, the temperature and/or the pH of a buffer in which is binding is occurring, e.g., by the addition of an elution buffer. Detergents, such as SDS, or denaturing agents such as urea or guanidine hydrochloride may also be used. Binding may be disrupted by adding an excess of competing binding molecules. Such binding is preferably by non-covalent bonds, such as hydrogen bonds, van der Waals forces, hydrophobic bonds or ionic bonds. Many binding moieties and the molecule to which each binding moiety binds are well known in the art, and the person skilled in the art is well versed in using binding moieties for purification purposes, such as in affinity columns.

Examples of binding moieties (and the substrate or binding partner to which they bind) that can be used in the present invention include, but are not limited to, biotin (streptavidin or anti-biotin antibody), a homopolymeric sequence, e.g., polyA (complementary homopolymeric sequence such as polydT), aptamers (aptamer target sequence), His-tags (divalent cations, usually on metal chelation resin), GST tag (glutathione), antibodies or antibody binding fragments, e.g., Fab fragments (specific antigens), peptides (specific antibodies), maltose binding protein (maltose), or any hydrophobic groups, which maybe purified using hydrophobic interaction and or reversed phase chromatography. The first and/or second substrates may be immobilised in a first and/or second column, beads, membranes or monoliths. Beads may be agarose, cellulose, silica, acrylic, polystyrene or plastic beads, as known in the art.

The appending of a first adaptor molecule to a linear double-stranded DNA molecule forms a linear DNA product precursor, which may be the same as the final purified linear DNA product, or it may be different. By linear DNA product precursor, it is meant a linear DNA product prior to contact with a first substrate and prior to obtaining the purified product. The purified linear DNA product may be the same as the linear DNA product precursor if the step of obtaining the purified product is performed by eluting the linear DNA product such that the binding moiety loses affinity for the substrate and the purified linear DNA product still comprises a first binding moiety. The purified linear DNA product may be different from the linear DNA product precursor if the step of obtaining the linear DNA product is performed by releasing the linear DNA product from the substrate by endonuclease digestion with a restriction endonuclease having a target sequence between the binding moiety and the first end of the linear region, i.e. the purified product will no longer comprise a first binding moiety.

The inventors have developed a method for purification of a linear DNA product that is nuclease resistant, surprisingly without interfering with the function of the nucleic acid product (e.g., it's use in therapeutic or research applications), in a simple step that eliminates unwanted material from a reaction volume (e.g., an aqueous volume in which the linear DNA molecule has been made, or an aqueous volume in which the adaptor molecule(s) have been appended to the linear DNA molecule). The inventors have unexpectedly discovered that the addition of a binding moiety to an adaptor molecule that is appended to a linear DNA product to confer nuclease resistance can also be used for the purification of the DNA product. The one-step appending of an adaptor molecule having dual function - conferring nuclease resistance and enabling purification - vastly simplifies the synthesis and purification of nuclease resistant linear DNA products.

The method of the invention may also comprise appending a second adaptor molecule to a second end of the linear region. The second adaptor molecule may comprise a second binding moiety. The first and/or second adaptor molecule may be a nucleic acid adaptor molecule, and the first and second adaptor molecule may be different. The first and second binding moiety may be different.

The first and second adaptor molecule may be identical or they may be different. For example, the first adaptor molecule and/or the second adaptor molecule may each comprise a hairpin. The first adaptor molecule and/or the second adaptor molecules may be double-stranded linear nucleic acid molecules each comprising one or more nuclease-resistant nucleotides. The first adaptor molecule may comprise a hairpin and the second adaptor molecule may be a double-stranded linear nucleic acid molecule comprising one or more nuclease-resistant nucleotides. Thus, the linear DNA product to be purified by the methods described herein may be resistant to nuclease (e.g. exonuclease) digestion.

The method of the invention may further comprise contacting the linear DNA product precursor with a second substrate under conditions in which the second binding moiety binds to the second substrate. The step of contacting the linear DNA product with a second substrate may be performed sequentially to the step of contacting the nucleic acid product with the first substrate, preferably after the step of washing the first substrate. Such sequential steps mean that any un-appended first adaptor molecules comprising a first binding moiety that may not be removed due to binding to the first substrate, will be removed after the linear DNA product precursor is contacted with the second substrate under conditions in which the second binding moiety binds to the second substrate. Linear DNA product precursor that binds to both the first and second substrates must therefore have both adapters (when the adapters, binding moieties and substrates are different) properly appended thereto.

The step of contacting the linear DNA product with a second substrate may be performed simultaneously with the step of contacting the nucleic acid product with the first substrate, such that the first end of the nucleic acid product (to which the first adaptor molecule comprising the first binding moiety is ligated) is bound to the first substrate and the second end of the nucleic acid product (to which the second adaptor molecule comprising the second binding moiety is ligated) is bound to the second substrate. Such simultaneous contacting steps means that any linear DNA product precursor comprising only either the first or second adaptor molecule may be bound, and all other unbound materials that may be present will be removed, such as enzymes, mononucleotides.

The use of a binding moiety and a substrate to which it binds (e.g., biotin and streptavidin) results in the immobilising of the DNA product precursor, such that linear DNA molecules that e.g. do not comprise adaptor molecules, will not be immobilised on the substrate and can be washed away using a suitable wash buffer. Two different binding moieties may be used (i.e., on a first and a second adaptor molecule) in order to separate the linear DNA product from e.g., un-appended adaptor molecules, or DNA products that have only one adaptor molecule ligated.

The first and/or second adaptor molecule may have a double stranded region and an overhang. The first and/or the second adaptor molecule may have a double stranded region and a blunt end. The first and/or the second adaptor molecule may have comprises double stranded region and a hairpin. The first adaptor molecule and/or the second adaptor molecule may comprise any one of the sequences of SEQ ID NO: 1, 2, 3, 4, 5, 6, 7, 8, 9, 10 a portion thereof. The first adaptor molecule and/or the second adaptor molecule may comprise at least 13, at least 14, at least 15, at least 16, at least 17, at least 18, or at least 19 contiguous nucleotides of SEQ ID NO: 1, 2, 3,4, 5, 6, 7, 8, 9, or 10. The first and second adaptor molecules may comprise an identical nucleic acid sequence. The first and second adaptor molecules may comprise a different nucleic acid sequence. The first adaptor molecule may comprise one or more protected nucleotides and the second adaptor molecule may comprise a hairpin or a stem loop region.

In particular, when the method of the invention is for purification of single stranded linear DNA product, the linear double-stranded region may comprise a first strand and an second strand, and the linear DNA product precursor may not comprise a binding moiety on the first strand, i.e., the strand of the first and/or second adaptor molecule that is appended to the first strand of the linear DNA product does not comprise a binding moiety, and the strand of the first and/or second adaptor molecule that is appended to the second strand of the linear DNA product precursor comprises a binding moiety. After the step of contacting the linear DNA product precursor with a first and/or second substrate, the linear DNA product precursor will be immobilised on the substrate by way of the binding moiety on the second strand. The first strand remains hybridised to the second strand. If the first strand is the linear DNA product to be purified, the step of obtaining the purified linear DNA product is performed under denaturing conditions, such that the two strands denature and the first strand is released.

Alternatively, if the second strand is the linear DNA product to be purified, the step of contacting the linear DNA product precursor with a first and/or second substrate may be performed under denaturing conditions, such that the first strand does not bind to the substrate and can be removed during the step of washing the first and/or second substrate. The first strand may be a sense strand. The second strand may be an antisense strand.

Alternatively, the linear double-stranded region may comprise a first strand and an second strand, and the linear DNA product precursor may not comprise a binding moiety on the second strand, i.e., the strand of the first and/or second adaptor molecule that is appended to the second strand of the linear DNA product does not comprise a binding moiety, and the strand of the first and/or second adaptor molecule that is appended to the first strand of the linear DNA product precursor comprises a binding moiety.

After the step of contacting the linear DNA product precursor with a first and/or second substrate, the linear DNA product precursor will be immobilised on the substrate by way of the binding moiety on the first strand. The second strand remains hybridised to the first strand. If the second strand is the linear DNA product to be purified, the step of obtaining the purified linear DNA product is performed under denaturing conditions, such that the two strands denature and the second strand is released whilst the first strand remains bound to the first and or second substrate. Alternatively, if the first strand is the linear DNA product to be purified, the step of contacting the linear DNA product precursor with a first and/or second substrate may be performed under denaturing conditions, such that the second strand does not bind to the substrate and can be removed during the step of washing the first and/or second substrate followed by elution of the first strand.

The method may further comprise, after the step of contacting the linear double stranded DNA molecule with a ligase and a first adaptor molecule, a step of nuclease digestion. The linear DNA product precursor may be treated with an exonuclease (for example, exonuclease III that cleaves 3' nucleotides, or exonuclease VII that cleaves both the 3' and 5' nucleotides, or exonuclease VIII that cleaves 5' end nucleotides, or exonuclease I to remove unprotected single stranded DNA) prior to the steps of contacting the linear DNA product precursor with a first substrate, such that any open ended, unprotected linear DNA molecules may be digested and also un-appended adaptor molecules comprising a binding moiety may be removed such that they do not compete with the linear DNA product precursor for binding to the substrate. Other exonucleases known in the art may be used. When the nuclease treatment is carried out prior to the purification method, the enzymes (nucleases) may also be removed during the method of purification of the linear DNA product. The nuclease digestion may be exonuclease digestion, such as exonuclease I and/or exonuclease III digestion. The step of nuclease digestion may take place before or after the step of contacting the linear DNA product precursor with a first substrate. The step of nuclease digestion may allow for removal of any double-stranded DNA molecules and/or adaptor molecules which have not been used to produce linear DNA products.

The linear double stranded region may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene.

The linear DNA product is preferably resistant to exonuclease digestion. When the linear DNA product is a double stranded DNA product, the linear double-stranded region may comprise at least two phosphorothioated nucleotides at internal positions in each strand, wherein the at least two phosphorothioated nucleotides at internal positions in each strand are selected from: a 5'-end nucleotide of the cassette; a 3'-end nucleotide of the cassette; and one or more nucleotides outside of the cassette. The cassette may comprise a coding sequence comprising a sense strand and an antisense strand, and wherein in the sense strand: one of the at least two phosphorothioated nucleotides is the 5'-end nucleotide of the cassette; and/or one of the at least two phosphorothioated nucleotides is in a first region of the sense strand, wherein the first region of the sense strand is 5' of the cassette; and one of the at least two phosphorothioated nucleotides is the 3'-end nucleotide of the cassette; and/or one of the at least two phosphorothioated nucleotides is in a second region of the sense strand, wherein the second region of the sense strand is 3' of the cassette.

When the linear DNA product is a single stranded product, the linear single stranded DNA product may be a protected DNA comprising a single-stranded DNA (ssDNA) cassette, wherein the protected DNA comprises x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1 and wherein the ssDNA cassette comprises at least 100 nucleotides.

In the method of the invention, the first adaptor molecule and/or the second adaptor molecule may be a synthetic adaptor molecule. The first adaptor molecule may comprise a hairpin and/or the second adaptor molecule may comprise a hairpin. The first adaptor molecule and/or the second adaptor molecule may comprise a single-stranded portion, optionally wherein: the single-stranded portion forms a hairpin; the single-stranded portion comprises less than 10, 9, 8, 7, 6, 5, 4, 3, 2 nucleotides; and/or the single-stranded portion comprises 5 nucleotides. In the method of the invention, the first adaptor molecule and/or the second adaptor molecule may comprise a double stranded portion, optionally wherein: the double-stranded portion comprises less than 50, 45, 40, 35, 30 base pairs; and/or the double-stranded portion comprises at least 10, 11, 12, 13, 14, 15 base pairs. In the method of the invention, the linear double-stranded region may be closed at a first end by the first adaptor molecule and may be closed at a second end by the second adaptor molecule. The first adaptor molecule may comprise one or more nuclease-resistant nucleotides (i.e. protected nucleotides) and the linear double-stranded region may be closed at the second end by the second adaptor molecule. The first and second adaptor molecules may comprise one or more nuclease-resistant nucleotides (i.e. protected nucleotides). The first adaptor molecule and/or the second adaptor molecule may comprise at least one phosphorothioated nucleotide. The linear DNA product may comprise at least 0.01%, at least 0.1%, at least 1% phosphorothioated nucleotides. The linear DNA product may comprise 1,2,3,4,5,6,7,8,9,10% phosphorothioated nucleotides.

The first adaptor molecule and/or the second adaptor molecule may comprise a hairpin loop such that ligation of the first or second adaptor molecule results in a closed first and/or second end, respectively, of the nucleic acid product. The hairpin loop may comprises a biotinylated nucleotide, an aptamer or a homopolymeric sequence. The first and/or the second adaptor molecule may have a double stranded region and a blunt end, or a double stranded region and an overhang. A first adaptor molecule and/or a second adaptor molecule may comprise a homopolymeric sequence or an aptamer and may not comprise a hairpin.

In the method of the invention, the first adaptor molecule and/or the second adaptor molecule may comprise at least one biotinylated nucleotide and the first and/or second substrate may comprise a biotin binding substrate, such as streptavidin or an anti-biotin antibody. The first adaptor molecule and/or the second adaptor molecule may comprise an aptamer and the first and/or second substrate, respectively, may comprise an aptamer target molecule specific for the aptamer. The first adaptor molecule and the second adaptor molecule may comprise different aptamers and the first and second substrate may comprise different aptamer target molecules. The first adaptor molecule and/or the second adaptor molecule may comprise a homopolymeric sequence, such as polyA, polyT, polyG or polyC and the first and/or second substrate may comprise an oligonucleotide comprising a complementary sequence.

In the method of the invention, the first adaptor molecule and/or the second adaptor molecule may comprise a histadine tag, comprising at least 3, at least 4, at least 5 or at least 6 histadine residues, and the first and/or second substrate may comprise a divalent metal ion, such as such as nickel, cobalt or iron immobilised on a metal chelating substrate. The first adaptor molecule and/or the second adaptor molecule may comprise a GST tag and the first and/or second substrate may comprise glutathione. The first and second adaptor molecules and the first and second substrate may comprise any binding moiety and substrate comprising a binding partner for the binding moiety, or substrate having affinity for the binding moiety.

In the method of the invention, the first adaptor molecule and/or second adaptor molecule may comprise a cleavable target sequence between the first and/or second end of the linear region and the binding moiety. The cleavable target sequence may be an endonuclease target sequence. Preferably, the endonuclease target sequence is a restriction endonuclease target sequence. Different restriction endonuclease target sequences are known to the skilled person. The cleavable target sequence may or may not be a Type IIS restriction endonuclease target sequence. The restriction endonuclease target sequence may or may not be a Bbsl, Bsal, BsmBI, BspQI, BtgZI, Esp3I,SapI, AarI, Acc36I, AclWI, Acul, Ajul, AloI, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV2I, Bsul, BtgZI, BtsCI, Btsl-v2, BtsMutl, Bvel, Csel, CspCI, Eam11041, Earl, Ecil, Ec031I, Eco57I, Esp3I, Faql, Faul, Fokl, Gsul, Hgal, Hphl, HpyAV, Lgul, Lmnl, Lsp1109I, LweI, Mboll, Mlyl, Mmel, Mnll, Mva12691, NmeAIII, PaqCI, PciSI, PctI, Plel, Ppsl, Psrl, Schl, SfaNI, Taqll, TspDTI and/or TspGWI target sequence.

The step of obtaining of the purified linear DNA product may be performed by releasing the linear DNA product from the substrate by endonuclease digestion with a restriction endonuclease having its cleavable target sequence in the first and/or second adaptor molecule. Preferably, the cleavable target sequence is located between the first and/or second binding moiety and the first and/or second end of the linear double stranded region. The cleavable target sequence may be located such that at least 15, 14, 13, 12, 11, 10, 9, 8, 6, 5, 4, 3, 2, or 1 nucleotides of the adaptor molecule remain appended to the double stranded target region, e.g., when the adaptor molecule comprises protected nucleotides that confer nuclease-resistance on the purified linear DNA product. The cleavable target sequence may be located directly adjacent to the end of the double stranded linear region of the linear DNA product precursor e.g., when the linear DNA product itself comprises protected nucleases and the adaptor molecule is used solely for purification reasons.

The step of obtaining the purified linear DNA product may be performed by releasing the linear DNA product from the first and/or second substrate under eluting conditions. By eluting conditions it is meant changing a ionic strength and/or pH and/or temperature and or polarity or hydrophobicity of the buffer in which the washing step is carried out, which maintains the binding of the binding moiety to the substrate, to a buffer which disrupts the affinity of the binding moiety to the substrate, and results in the releasing of the linear DNA product (via the release of the binding moiety) from the substrate. Release of the binding moiety from the substrate may also be performed by adding an excess of free binding moiety to the substrate, such that it competes with the binding moiety of the linear DNA product precursor such that the affinity for the first or second binding moiety for the first or second substrate is reduced or disrupted and the linear DNA product is released. The skilled person is aware of which eluting conditions are suitable for a particular binding moiety and substrate to which it is bound. Eluting buffers for particular binding moieties are well known in the art.

The appending of the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The appending of the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region. The hybridization is based on complementarity of a portion of the first and/or second adaptor molecules to the first and/or second end of the linear double-stranded region.

The first and/or second adaptor molecules may comprise a nucleic acid sequence. The first and second adaptor molecules may comprise an identical nucleic acid sequence. The first and second adaptor molecules may comprise different nucleic acid sequences.

The first adaptor molecule may comprise a portion that is complementary to the first end of the linear double-stranded region (or the linear portion of the double-stranded DNA molecule). The second adaptor molecule may comprise a portion that is complementary to the second end of the linear double-stranded region (or the linear portion of the double-stranded DNA molecule). The first adaptor molecule may comprise a portion that anneals to the first end of the linear double-stranded region (or the linear portion of the double-stranded DNA molecule). The second adaptor molecule may comprise a portion that anneals to the second end of the linear double-stranded region (or the linear portion of the double-stranded DNA molecule). The first adaptor molecule may comprise a portion that is complementary and anneals to the first end of the linear double-stranded region (or the linear portion of the double-stranded DNA molecule). The second adaptor molecule may comprise a portion that is complementary and anneals to the second end of the linear double-stranded region (or the linear portion of the double-stranded DNA molecule).

The portion that is complementary or anneals to the first or second end of the linear double-stranded region (or the linear portion of the double-stranded DNA molecule) may be a 5' overhang or a 3' overhang of the first and/or second adaptor molecule. The overhang of the first adaptor molecule may be complementary to the first end of the linear double-stranded region and/or the overhang of the second adaptor molecule may be complementary to the second end of the double-stranded region. The overhang of the first adaptor molecule may anneal to the first end of the linear double-stranded region and/or the overhang of the second adaptor molecule may anneal to the second end of the linear double-stranded region. The overhang of the first adaptor molecule may be complementary to and anneal to the first end of the linear double-stranded region and/or the overhang of the second adaptor molecule may be complementary to and anneal to the second end of the linear double-stranded region.

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The adaptor molecule may be a nucleic acid adaptor molecule. The adaptor molecule may be double-stranded. The adaptor molecule may comprise a portion that is double-stranded. One or both strands of a double stranded portion of an adaptor molecule may comprise a binding moiety.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to nuclease digestion (e.g. exonuclease III digestion). For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The adaptor molecule may comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be at least 10, at least 20, at least 30, at least 40, at least 50 nucleotides in length. Preferably, the homopolymeric sequence is at least 20 nucleotides in length. More preferably still, the homopolymeric sequence is 12, 13, 14, 15, 16, 17, 18, 19, 20, 21, 22, 23, 24, 25, 26, 27, 28, 29 or 30 nucleotides in length. The homopolymeric sequence may comprise a polyA sequence of at least 12 nucleotides, such that it binds to a oligo-dT purification column, as known by the skilled person.

The method may comprise, before (a) (i.e. the step of contacting the double-stranded DNA molecule with the ligase and the first adaptor molecule), contacting the linear DNA molecule with an endonuclease to create overhanging ends, such that a first adaptor molecule is able to be appended by e.g. hybridisation and/or ligation. The step of contacting with an endonuclease to create overhanging ends may be carried out simultaneously with the contacting with a ligase and a first adaptor molecule.

The method may comprise, before step (a) (i.e. the step of contacting the double-stranded DNA molecule with the ligase and the first adaptor molecule), a step of amplification of a DNA template molecule to produce the double-stranded DNA molecule. Thus, the invention may provide a method for purification of a linear DNA product, comprising the steps of:
(a) amplification of a DNA template molecule comprising at least one cleavable (e.g. endonuclease) target sequence to generate a linear DNA molecule;
(b) contacting the double-stranded DNA molecule with an endonuclease, a ligase and a first adaptor molecule to form a linear DNA product precursor comprising a linear region and a first adaptor molecule appended to a first end of the linear region, wherein the first adaptor molecule comprises a first binding moiety;
(c) contacting the linear DNA product precursor with a first substrate under conditions in which the first binding moiety binds to the first substrate;
(d) washing the first substrate to remove unbound linear DNA molecules; and
(e) obtaining a purified linear DNA product.

The cleavable endonuclease target sequence present in the DNA template and the cleavable endonuclease target sequence that may be present in the first adaptor molecule (and/or a second adapter molecule) may be different, such that the first and/or second) adaptor molecule is not cleaved during step (b).

Also included in the invention is a linear DNA product precursor comprising a double stranded linear region, and a first adaptor molecule appended to a first end of the product precursor, wherein the first adaptor molecule comprises a first binding moiety. The linear DNA product precursor may also comprise a second adaptor molecule appended to a second end of the product precursor. The second adaptor molecule may also comprise a binding moiety.

Also included in the invention is a linear DNA product precursor comprising a double stranded linear region, and a first adaptor molecule appended to a first end of the product precursor, wherein the first adaptor molecule comprises a first binding moiety. The linear DNA product precursor may also comprise a second adaptor molecule appended to a second end of the product precursor. The second adaptor molecule may also comprise a binding moiety.

### Linear DNA molecules having nuclease resistance

PCT/EP2022/061630 describes the use of internally positioned phosphorothioated nucleotides within a linear double stranded DNA product in order to confer nuclease-resistance on the DNA product. In order to purify a nuclease resistant DNA product comprising internally positioned phosphorothioated nucleotides, a first adaptor molecule comprising a fist binding moiety may be appended to a first end. A second adaptor molecule comprising a second binding moiety may be appended to a second end.

Therefore, prior to step of the method of the invention of contacting a DNA molecule with a ligase and a first adaptor molecule, the method of purification of a linear DNA product may comprise the steps of:
(i) rolling circle amplification of a DNA template molecule comprising at least one endonuclease target sequence in the presence of at least one type of a phosphorothioated nucleotide to generate a linear double-stranded DNA region; and
(ii) contacting the double-stranded DNA region with at least one endonuclease to generate a linear double-stranded DNA molecule;

Therefore, the method of purification of a linear double-stranded DNA product with enhanced resistance to nuclease digestion may comprise the steps of:
(a) rolling circle amplification of a DNA template molecule comprising at least one endonuclease target sequence in the presence of at least one type of a phosphorothioated nucleotide to generate a double-stranded DNA region;
(b) contacting the double-stranded DNA product with at least one endonuclease to generate a linear double-stranded DNA molecule;
(c) contacting the double-stranded DNA molecule with a ligase and a first and/or second adaptor molecule to form a linear DNA product precursor comprising a linear double stranded region and, a first and/or second adaptor molecule appended to a first and/or second end of the linear region, wherein the first and/or second adaptor molecule comprises a first and/or second binding moiety
(d) contacting the double-stranded DNA product with a first and/or second substrate under conditions in which the first and/or second binding moiety binds to the first and/or second substrate;
(e) washing the first and/or second substrate to remove any unbound linear double-stranded DNA molecules; and
(g) obtaining the purified linear DNA product.

The first and/or second comprise a target sequence for an endonuclease between the step of obtaining the purified DNA product may be performed by endonuclease digestion with a restriction endonuclease having a target sequence in the first and/or second adaptor molecule. The target sequence may be located such that the first and/or second adaptor molecule is completely removed by cleaving with the endonuclease from the purified linear DNA product, i.e., the purified DNA product is the same as the double stranded DNA product prior to the appending of a first and/or second adaptor molecule. Alternatively, the endonuclease may cleave only a portion of the adaptor molecule from the linear DNA product, such that 1, 2, 3, 4, 5, 6, 7, 8, 9, 10, 11, or 12 base pairs of the first and/or second adaptor molecule remain.

As an alternative, or in addition to a linear region comprising internally positioned phosphorothioated nucleotides, nuclease-resistance may be conferred by one or more adaptor molecules.

PCT/EP2022/071413 describes methods of making linear DNA products having nuclease-resistance. Methods of purification of such linear DNA products are provided by the invention.

### Partially closed DNA product

The DNA product to be purified may be a partially closed linear DNA product. A partially closed DNA product to be purified may be produced by a method comprising the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, and wherein the first adaptor molecule is appended to a first end of the linear double-stranded region and the second adaptor molecule is appended to a second end of the linear double-stranded region, and wherein the first adaptor molecule is a nucleic acid molecule that comprises one or more nuclease-resistant nucleotides (i.e. protected nucleotides) and wherein the linear double-stranded region is closed at the second end by the second adaptor molecule and wherein the first and/or second adaptor molecule comprises a first and/or second binding moiety.

A method of purification of a partially closed linear DNA product may, therefore, comprise the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a single contiguous aqueous volume to form a linear DNA product precursor, which is a partially closed linear DNA product precursor comprising a linear double-stranded region and a first adaptor molecule appended to a first end of the linear double-stranded region and a second adaptor molecule appended to a second end of the linear double-stranded region, and wherein the first adaptor molecule is a nucleic acid molecule that comprises one or more nuclease-resistant nucleotides (i.e. protected nucleotides) and wherein the linear double-stranded region is closed at the second end by the second adaptor molecule and wherein the first and/or second adaptor molecule comprises a first and/or second binding moiety;
(c) contacting the double-stranded DNA product precursor with a first substrate under conditions in which the first binding moiety binds to the first substrate;
(d) washing the first substrate to remove any unbound linear double-stranded DNA molecules; and
(f) obtaining the purified linear DNA product, which is a partially closed linear DNA product.

The invention provides a partially closed linear DNA product precursor comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprise one or more nuclease-resistant nucleotides and a first binding moiety, and wherein the linear portion of the double-stranded DNA molecule is closed at the second end by the second adaptor molecule. Thus, the invention provides a partially closed linear DNA product precursor which is closed (or covalently closed) at a second end and open at a first end and comprises a first binding moiety. The partially closed linear DNA product precursor comprises one or more nuclease-resistant nucleotides in the open-end region and/or at the first end. The open-end region may be at the 3' end or 5' end of the molecule.

The invention provides a partially closed linear DNA product precursor comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprise one or more nuclease-resistant nucleotides, and wherein the linear portion of the double-stranded DNA molecule is closed at the second end by the second adaptor molecule, which comprises a second binding moiety. Thus, the invention provides a partially closed linear DNA product precursor which is closed (or covalently closed) at a second end and open at a first end and comprises a second binding moiety. The partially closed linear DNA product precursor comprises one or more nuclease-resistant nucleotides in the open-end region and/or at the first end. The open-end region may be at the 3' end or 5' end of the molecule.

The invention provides a partially closed linear DNA product precursor comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprise one or more nuclease-resistant nucleotides and a first binding moiety, and wherein the linear portion of the double-stranded DNA molecule is closed at the second end by the second adaptor molecule, which comprises a second binding moiety. Thus, the invention provides a partially closed linear DNA product precursor which is closed (or covalently closed) at a second end and open at a first end and comprises a first and a second binding moiety. The partially closed linear DNA product precursor comprises one or more nuclease-resistant nucleotides in the open-end region and/or at the first end. The open-end region may be at the 3' end or 5' end of the molecule.

The open-end region refers to at least 5, at least 10, at least 15, or at least 20 base pairs located closest to the open end of the DNA product precursor. The partially closed linear DNA product precursor may comprise one or more nuclease-resistant nucleotides in a sense and/or an antisense strand. Thus, for example, one or more nucleotides of the 20 base pairs located closest to the open end of the partially closed linear DNA product precursor may be a nuclease-resistant nucleotide. Preferably, the partially closed linear DNA product precursor comprises at least 5 nuclease-resistant nucleotides in the open-end region.

The partially closed linear DNA product precursor may comprise a double-stranded DNA portion that is closed at a first end and open at a second end. The partially closed linear DNA product precursor may comprise a double-stranded DNA portion that is closed at a first end by a single-stranded portion (i.e. it may comprise a first hairpin at the first end) and open at a second end. The partially closed linear DNA product precursor may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA product precursor. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise two or more, three or more, four or more, or five or more nuclease-resistant nucleotides in both the sense and antisense strand. Preferably, the open-end region adjacent to the second end comprises five nuclease-resistant nucleotides in both the sense and antisense strand.

The partially closed linear DNA product precursor may comprise a hairpin-loop at the 5' end or the 3' end. The partially closed linear DNA product precursor may comprise a first adaptor molecule at a first end and a second adaptor molecule at a second end. The first adaptor molecule may comprise a hairpin and a second adaptor molecule may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The partially closed linear DNA product precursor may be a DNA molecule having a double-stranded portion closed at a first end by ligation of a first adaptor molecule (e.g. hairpin adaptor molecule) to the first end and comprising at a second end a double stranded linear adaptor molecule comprising protected nucleotides (i.e. nucleotides resistant to nuclease (e.g. exonuclease) digestion). The partially closed linear DNA product precursor may comprise a binding moiety on the closed or on the open end, such as a biotinylated nucleotide. The biotinylated nucleotide may be dT, dA, dG or dC.

The partially closed linear DNA product precursor may comprise (i) a cassette, wherein the cassette comprises a sense strand and an antisense strand; and (ii) one or more nuclease-resistant nucleotides in an open-end region of the partially closed linear DNA product precursor, wherein the open-end region is 5' of the sense strand of the cassette. The partially closed linear DNA product precursor may comprise (i) a cassette, wherein the cassette comprises a sense strand and an antisense strand; and (ii) one or more nuclease-resistant nucleotides in an open-end region of the partially closed linear DNA product precursor, wherein the open-end region is 5' of the antisense strand of the cassette. The partially closed linear DNA product precursor may comprise (i) a cassette, wherein the cassette comprises a sense strand and an antisense strand; and (ii) one or more nuclease-resistant nucleotides in an open-end region of the partially closed linear DNA product precursor, wherein the open-end region is 3' of the sense strand of the cassette. The partially closed linear DNA product precursor may comprise (i) a cassette, wherein the cassette comprises a sense strand and an antisense strand; and (ii) one or more nuclease-resistant nucleotides in an open-end region of the partially closed linear DNA product precursor, wherein the open-end region is 3' of the antisense strand of the cassette.

The partially closed linear DNA product precursor may comprise (i) a cassette, wherein the cassette comprises a sense strand and an antisense strand; (ii) one or more nuclease-resistant nucleotides in an open-end region of the partially closed linear DNA product precursor, wherein the open-end region is 5' of the sense strand of the cassette; and (iii) one or more nuclease-resistant nucleotides in an open-end region of the partially closed linear DNA product precursor, wherein the open-end region is 3' of the antisense strand of the cassette.

The partially closed linear DNA product precursor may comprise (i) a cassette, wherein the cassette comprises a sense strand and an antisense strand; (ii) one or more nuclease-resistant nucleotides in an open-end region of the partially closed linear DNA precursor, wherein the open-end region is 3' of the sense strand of the cassette; and (iii) one or more nuclease-resistant nucleotides in an open-end region of the partially closed linear DNA product precursor, wherein the open-end region is 5' of the antisense strand of the cassette.

The adaptor molecule comprising a hairpin (closed end) comprising a binding moiety may also comprise protected nucleotides between the hairpin and the open end of the adaptor molecule that is ligated to the double-stranded linear region of the linear DNA product precursor. The adaptor molecule may also comprise an endonuclease target sequence between the hairpin and the protected nucleotides, such that the partially closed linear DNA product precursor may be released from the substrate using a endonuclease cleaving at the target sequence, resulting in a purified linear DNA product with two open ends and resistant to exonuclease digestion.

### Open ended DNA product

When the linear DNA product to be purified is an open ended DNA product comprising nuclease resistant nucleotides, to confer nuclease resistance, the linear open ended DNA molecule may be produced by a method comprising the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a single contiguous aqueous volume; and
(c) incubating the single contiguous aqueous volume to generate the partially closed linear DNA product, wherein the open ended linear DNA product comprises a linear double-stranded region, wherein the linear double-stranded region comprises a linear portion of the double-stranded DNA molecule, and wherein the first and second adaptor molecules are nucleic acid molecules that comprise one or more nuclease-resistant nucleotides (i.e. protected nucleotides) and wherein the first and/or second adaptor molecule comprises a first and/or second binding moiety.

Therefore, a method of purification of an open ended linear DNA molecule may comprise the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a linear DNA product precursor, which is an open ended linear DNA product precursor comprising a linear double-stranded region and a first adaptor molecule appended to a first end of the linear double-stranded region and a second adaptor molecule appended to a second end of the linear double-stranded region, and wherein the first and second adaptor molecules are nucleic acid molecules that comprise one or more nuclease-resistant nucleotides (i.e. protected nucleotides) and wherein the first and/or second adaptor molecule comprises a first and/or second binding moiety;
(c) contacting the double-stranded DNA product precursor with a first substrate under conditions in which the first binding moiety binds to the first substrate;
(d) washing the first substrate to remove any unbound linear double-stranded DNA molecules; and
(f) obtaining the purified open ended linear DNA product.

The first or second adaptor molecule may comprise a cleavable target sequence for an endonuclease such that the open ended DNA product may be released from a substrate using an endonuclease that cleaves at the target sequence. The target sequence may be between the binding moiety and the protected nucleotides, such that the purified open ended linear DNA product comprises protected nucleotides and is resistant to nucleases.

The invention provides a linear open ended DNA product precursor comprising a linear double-stranded region, wherein the linear double-stranded region comprises a linear portion of a double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides, and the first adaptor molecule comprises a binding moiety.

The invention provides a linear open ended DNA product precursor comprising a linear double-stranded region, wherein the linear double-stranded region comprises a linear portion of a double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides, and the second adaptor molecule comprises a binding moiety.

The invention provides a linear open ended DNA product precursor comprising a linear double-stranded region, wherein the linear double-stranded region comprises a linear portion of a double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides, and the first and second adaptor molecule comprise a first and/second binding moiety.

### Closed linear DNA product

The linear DNA product to be purified may be a closed linear DNA product, e.g., a covalently closed DNA product. If the linear DNA product to be purified is a closed linear DNA product, it may be produced by any method described in PCT/EP2022/071413. For example, a closed linear DNA product may be produced by a method comprising the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a single contiguous aqueous volume; and
(c) incubating the single contiguous aqueous volume to generate the partially closed linear DNA product, wherein the partially closed linear DNA product comprises a linear double-stranded region, wherein the linear double-stranded region comprises a linear portion of the double-stranded DNA molecule, , and wherein the linear double-stranded region is closed at a first end by the first adaptor molecule and closed at a second end by the second adaptor molecule and wherein the first and/or second adaptor molecule comprises a first and/or second binding moiety.

A method of purification of a closed linear DNA product may, therefore, comprise the steps of:
(a) amplifying a DNA template molecule comprising at least one cleavable (e.g. endonuclease) target sequence to generate a double-stranded DNA molecule;
(b) contacting a double-stranded DNA molecule with an endonuclease, a ligase and first and second adaptor molecules to form a single contiguous aqueous volume to form a linear DNA product precursor, which is a closed linear DNA product precursor comprising a linear double-stranded region and a first adaptor molecule appended to a first end of the linear double-stranded region and a second adaptor molecule appended to a second end of the linear double-stranded region, and , and wherein the linear double-stranded region is closed at a first end by the first adaptor molecule and closed at a second end by the second adaptor molecule and wherein the first and/or second adaptor molecule comprises a first and/or second binding moiety;
(c) contacting the double-stranded DNA product precursor with a first substrate under conditions in which the first binding moiety binds to the first substrate;
(d) washing the first substrate to remove any unbound linear double-stranded DNA molecules; and
(f) obtaining the purified linear DNA product, which is a closed linear DNA product.

The closing of the linear double-stranded region by the first adaptor molecule and/or the second adaptor molecule may be performed by hybridization or ligation of the adaptor molecules to the ends of the linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The closing of the linear double-stranded region by the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region.

Both the first and second adaptor molecules used in the methods described herein may comprise a self-complementary element which creates a loop, such as a hairpin loop or a stem loop. Thus, the one of the adaptor molecules may comprise a hairpin or a stem-loop. The adaptor molecule may comprise a double-stranded portion comprising a sense strand and an antisense strand, wherein the sense strand and the antisense strand are linked together in a hairpin such that the sense strand is hybridized to the antisense strand. The double-stranded portion of an adaptor molecule may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the adaptor molecule (e.g. the overhang) may be complementary to the first end or the second end of the linear double-stranded region.

The double stranded portion of the adaptor molecule may comprise a binding moiety. The hairpin or stem loop single stranded portion may comprise a binding moiety, such as one or more biotinylated nucleotides. The biotinylated nucleotides may be dT, dA, dC, and/or dG.

The adaptor molecule comprising a hairpin comprising a binding moiety may also comprise protected nucleotides. An adaptor molecule comprising a hairpin may also comprise an endonuclease target sequence. An adaptor molecule may comprise a hairpin, protected nucleotides and an endonuclease target sequence. The hairpin may or may not comprise a homopolymeric sequence, or an aptamer.

The invention also provides a closed linear DNA product precursor comprising a linear double-stranded region, wherein the linear double-stranded region comprises a linear portion of a double-stranded DNA molecule, and wherein the linear double-stranded region is closed at a first end by a first adaptor molecule and closed at a second end by the second adaptor molecule, wherein the first adaptor molecule comprises a binding moiety.

The invention also provides a closed linear DNA product precursor comprising a linear double-stranded region, wherein the linear double-stranded region comprises a linear portion of a double-stranded DNA molecule, and wherein the linear double-stranded region is closed at a first end by a first adaptor molecule and closed at a second end by the second adaptor molecule, wherein the second adaptor molecule comprises a binding moiety.

The invention also provides a closed linear DNA product precursor comprising a linear double-stranded region, wherein the linear double-stranded region comprises a linear portion of a double-stranded DNA molecule, and wherein the linear double-stranded region is closed at a first end by a first adaptor molecule and closed at a second end by the second adaptor molecule, wherein the first adaptor molecule comprises a binding moiety and the second adaptor molecule comprises a second binding moiety.

### Single-stranded DNA products

The invention also provides a method for purification of a linear single stranded DNA product, wherein the method comprises:
(a) contacting a linear double-stranded DNA molecule with a ligase and a first adaptor molecule, to form a linear DNA product precursor comprising a linear double-stranded region comprising a first strand and a second strand, and a first adaptor molecule appended to a first end of the linear region, wherein the first adaptor molecule comprises a first binding moiety, and the linear DNA product precursor does not comprise a binding moiety on the first strand;
(b) contacting the linear DNA product precursor with a first substrate under conditions in which the first binding moiety binds to the first substrate;
(c) washing the first substrate to remove unbound linear double-stranded DNA molecules; and
(d) obtaining a purified linear DNA product.

A method of the invention for the purification of a single stranded linear DNA product may also comprise:
(a) contacting a linear double-stranded DNA molecule with a ligase and a first adaptor molecule, to form a linear DNA product precursor comprising a linear double-stranded region comprising a first strand and a second strand, and a first adaptor molecule appended to a first end of the linear region, wherein the first adaptor molecule comprises a first binding moiety, and the linear DNA product precursor does not comprise a binding moiety on the second strand;
(b) contacting the linear DNA product precursor with a first substrate under conditions in which the first binding moiety binds to the first substrate;
(c) washing the first substrate to remove unbound linear double-stranded DNA molecules; and
(d) obtaining a purified linear DNA product.

The first adaptor molecule may comprise x protected nucleotides and the second adaptor molecule may comprise y protected nucleotides. The first and second adaptor molecules may comprise a binding moiety only on the first strand, i.e., the first and second adaptor molecules may not comprise a binding moiety on the second strand. The first and second adaptor molecules may comprise a binding moiety only on the second strand, i.e., the first and second adaptor molecules may not comprise a binding moiety on the first strand. The first and/or second adaptor molecule may comprise protected nucleotides on the opposite strand that comprises the binding moiety, i.e. the first strand may comprise a binding moiety and the second strand may comprise protected nucleotides or vice versa. The first and second adaptor molecules may comprise protected nucleotides and a binding moiety on the same strand. The first and second adaptor molecules may comprise protected nucleotides on both strands.

The double stranded DNA molecule may be produced by a method comprising:
(a) amplifying a DNA template to generate a linear double stranded DNA molecule, wherein the DNA template comprises an endonuclease target sequence, optionally wherein the DNA template is amplified by rolling circle amplification.

Therefore, the method for purification of a ssDNA product may comprise:
(a) amplifying a DNA template to generate a double stranded DNA, wherein the DNA template comprises an endonuclease target sequence, optionally wherein the DNA template molecule is amplified by rolling circle amplification;
(b) contacting the linear double-stranded DNA molecule with an endonuclease, a ligase and a first adaptor molecule, to form a linear DNA product precursor comprising a linear double-stranded region comprising a first strand and a second strand, and a first adaptor molecule appended to a first end of the linear region, wherein the first adaptor molecule comprises a first binding moiety, and the linear DNA product precursor does not comprise a binding moiety on the first strand;
(c) contacting the linear DNA product precursor with a first substrate under conditions in which the first binding moiety binds to the first substrate;
(d) washing the first substrate to remove unbound linear double-stranded DNA molecules; and
(e) obtaining a purified linear DNA product.

The step of obtaining the purified single stranded linear DNA product may be performed under denaturing conditions, such that the second strand comprising a binding moiety remains bound to the substrate and the first strand that does not comprise a binding moiety is released to obtain the purified single stranded linear DNA product (the first strand). The step of contacting the linear DNA product precursor with a first substrate may be performed under native conditions, such that the first strand remains hybridised to the second strand until denaturing conditions are introduced at the step of obtaining the purified single stranded linear DNA product.

In some cases, both strands may be separately obtained: the first strand by performing the step of washing the first substrate under denaturing conditions such that the first strand is no longer hybridised to the second strand and can be obtained in a flow through or supernatant, whilst the second strand remains bound to the substrate; and the second strand by performing the obtaining step under conditions that elute the binding moiety from the substrate, i.e., disrupt the binding of the binding moiety to the substrate and obtain the second strand.

Alternatively, the step of contacting the linear DNA product precursor may be performed under denaturing conditions, such that only the second strand comprising the binding moiety binds to the substrate (as a single strand), and the first strand that does not comprise a binding moiety flows through or remains in the supernatant. The first strand may be obtained from the flow through or supernatant or may be discarded. The second strand may be obtained under eluting conditions that disrupt the binding between the first binding moiety and the substrate. The second strand may be obtained by cleaving the single strand from the first binding moiety.

The method of purification of a single stranded linear DNA product may comprise:
(a) amplifying a DNA template to generate a double stranded DNA, wherein the DNA template comprises an endonuclease target sequence, optionally wherein the DNA template molecule is amplified by rolling circle amplification;
(b) contacting the linear double-stranded DNA molecule with an endonuclease, a ligase and a first adaptor molecule, to form a linear DNA product precursor comprising a linear double-stranded region comprising a first strand and a second strand, and a first adaptor molecule appended to a first end of the linear region, wherein the first adaptor molecule comprises a first binding moiety, and the linear DNA product precursor does not comprise a binding moiety on the second strand;
(c) contacting the linear DNA product precursor with a first substrate under conditions in which the first binding moiety binds to the first substrate;
(d) washing the first substrate to remove unbound linear double-stranded DNA molecules; and
(e) obtaining a purified linear DNA product.

The invention provides a protected or partially protected linear DNA product precursor comprising a double stranded linear region comprising a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1, and wherein a first adaptor molecule is appended to a first end of the double stranded linear region, and wherein the first adaptor molecule comprises a binding moiety on the first strand and does not comprise a binding moiety on the second strand. The second strand may comprise protected nucleotides. The second strand may not comprise protected nucleotides.

The invention provides a protected or partially protected linear DNA product precursor comprising a double stranded linear region comprising a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1, and wherein a first adaptor molecule is appended to a first end of the double stranded linear region, and wherein the first adaptor molecule comprises a binding moiety on the second strand and does not comprise a binding moiety on the first strand. The second strand may comprise protected nucleotides. The second strand may not comprise protected nucleotides.

The invention provides a protected or partially protected linear DNA product precursor comprising a double stranded linear region comprising a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1, and wherein a first adaptor molecule is appended to a first end of the double stranded linear region, and a second adaptor molecule is append to a second end of the double stranded region, wherein the first adaptor molecule comprises a binding moiety on the first strand and does not comprise a binding moiety on the second strand, and wherein the second adaptor molecule comprises a binding moiety on the first strand and does not comprise a binding moiety on the second strand. The second strand may comprise protected nucleotides. The second strand may not comprise protected nucleotides.

The invention provides a protected or partially protected linear DNA product precursor comprising a double stranded linear region comprising a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1, and wherein a first adaptor molecule is appended to a first end of the double stranded linear region, and a second adaptor molecule is append to a second end of the double stranded region, wherein the first adaptor molecule comprises a binding moiety on the second strand and does not comprise a binding moiety on the first strand, and wherein the second adaptor molecule comprises a binding moiety on the second strand and does not comprise a binding moiety on the first strand. The second strand may comprise protected nucleotides. The second strand may not comprise protected nucleotides.

The step of contacting the substrate may be performed under denaturing conditions such that the strand that does not comprise the binding moiety does not bind and is removed in the washing step. The strand comprising the binding moiety is later released to obtain the purified single stranded linear DNA product.

The closed linear DNA product precursor, purified linear DNA product precursor or purified partially closed linear DNA product precursor or a partially protected or protected linear DNA product precursor may comprise a cassette, optionally a single cassette. The linear portion of a double-stranded DNA molecule (of a closed linear DNA product, linear DNA product or partially closed linear DNA product) may comprise a cassette, optionally a single cassette. Accordingly, the cassette (or single cassette) is located between the first and second adaptor molecules.

The term "single cassette" as used herein is intended to encompass a molecules that do not comprise or consist of a plurality of cassettes. That is to say that the closed linear DNA product precursor, linear DNA product precursor, or partially closed linear DNA product precursor, may comprise only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

### Binding Moieties

### Biotin

Biotin is used widely in protein purification and nucleic acid purification methods, due to the highly specific and high affinity between biotin and streptavidin. As an alternative to streptavidin, an anti-biotin antibody may be used.

Biotinylated nucleotides can be incorporated into any nucleic acid sequence. Typically biotinylated Thymidine is used, but other nucleotides may also be biotinylated. The biotinylated nucleic acid can be purified using affinity chromatography together with a column or beads having immobilised streptavidin (or avidin). The high affinity between biotin and streptavidin means that although washing away any unbound product can be carried out in nearly any conditions, including high salt (up to 5M), high temperature (up to 70°C) or alkaline conditions (above pH8.0), it is also required to use stronger conditions than other purification methods to elute biotin containing molecules from streptavidin substrate, e.g., 6M GuHCl, or pH4.0 and lower, or 80°C water.

Increasing the number of biotin molecules in sequence can increase binding strength with streptavidin.

A cleavable target sequence may be included in an adaptor molecule sequence between the DNA product and the biotinylated nucleotides, such that the DNA product may be eluted without having to use harsh elution conditions.

### Homopolymeric sequence

A homopolymeric sequence, such as a polyA sequence in or at the end of the adaptor molecule sequence, can be used to purify the linear DNA product by taking advantage of the complementarity of nucleotides. A polyA sequence, for example, will hybridise to a polyT sequence. The skilled person is well aware of the use of polydT columns to purify mRNA and other molecules comprising a polyA sequence.

For example, a polydT oligonucleotide (10 to 20 residues) may be immobilised on magnetic beads, agarose or sepharose beads or on a column (e.g., sepharose) or other immobilising medium. Contacting the bead or column (or other immobilising medium) with a linear DNA product precursor comprising a polyA sequence under the correct conditions to encourage hybridisation between the polydT and the polyA sequence of the DNA product, may be carried out under conditions such that the DNA product precursor binds to substrate. Conditions may be created using a suitable buffer (may be referred to as a equilibration buffer or an application buffer), such as 50 mM phosphate, 250 mM NaCl, 2 mM EDTA, pH 7.0. High salt conditions may include a salt concentration of 0.25M to 0.5M NaCl or KCI, and 150to 300mM phosphate. The skilled person is aware of the conditions required and the conditions are well known and well documented in the art.

The column or beads may be washed in a buffer that encourages the maintenance of the hybridisation and at the same time disrupts any non-specific binding. A wash buffer may comprise, for example, 50 mM phosphate, 2 mM EDTA, pH 7.0. Elution may be carried out in a buffer such as 10 mM Tris, pH 7.0. The polyA sequence of the linear DNA product precursor interacts with substrate comprising oligo dT ligands in higher salt loading conditions (relative to the elution buffer), where electrostatic repulsion between negatively charged backbones of both polyA nucleotides and the rest of sequence of the DNA product, and oligo dT are reduced, and H-bonding in the T-A base pair is encouraged.

### His Tag purification

A linear DNA product precursor may comprise a histidine tag, comprising at least 3, at least4, st least 5 or at least 6 histidine residues. A tag may be attached to one or both strands of the linear DNA product precursor by way of a spacer or a linker. Supports such as beaded agarose or magnetic particles can be associated with chelating groups to immobilize the desired metal ions, which then function as ligands for binding and purification of polyhistidine affinity-tagged molecules. This may be known as immobilized metal affinity chromatography and is a widely-used method for rapidly purifying molecules with histidine tags.

The chelators most commonly used and known to the skilled person are nitrilotriacetic acid (NTA) and iminodiacetic acid (IDA). Once a substrate, such as IDA-agarose or NTA-agarose resin, is prepared, it can be "loaded" with the desired divalent metal (e.g., Ni, Co, Cu, and Fe).

A typical binding/wash buffer consists of Tris-buffer saline (TBS), pH 7.2, containing 10-25 mM imidazole in which the step in which the linear DNA product precursor may be contacted with the substrate. The step was washing the substrate to remove any unbound linear double stranded DNA molecules may also be carried out in the same buffer. The low-concentration of imidazole helps to prevent nonspecific binding.

Elution and recovery of captured His-tagged protein from a substarte maybe performed by using a high concentration of imidazole (at least 200 mM), a low pH (e.g., 0.1 M glycine-HCI, pH 2.5) or an excess of strong chelators (e.g., EDTA). Imidazole is the most common elution agent, as known in the art.

A His tag (e.g., 6x histidine residues) may be attached to a first or second adaptor molecule via a linker. A linker may be a short (3 to 8) residue polypeptide, or may be directly conjugated to an end of an adaptor molecule.

### Aptamers

Aptamers are artificially selected ligands that specifically bind to a target molecules, such as integrin, a cell receptor or adhesion molecules. The use of aptamers allow purification by combing a particular aptamer with its target ("epitope"), immobilised on a substrate. They are single-stranded oligonucleotides that can binding to various small molecules with high specificity and affinity. Binding, washing and elution conditions will vary, depending on the aptamer /target combination. The skilled person is able to determine such conditions with minimal trial and error, e.g. varying salt concentration or pH.

### Other methods

The invention also provides a method for concentration of a linear DNA product comprising the steps of:
(a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
(b) contacting the linear DNA product precursor with a first substrate under conditions in which the first binding moiety binds to the first substrate in a buffer volume;
(c) eluting the bound linear DNA product in a lower buffer volume; and
(d) obtaining a concentrated linear DNA product.

Also provided is a method of exchanging a buffer composition of a buffer volume comprising a linear DNA product, wherein the method comprises the steps of:
(a) contacting the buffer volume comprising a linear double-stranded DNA molecule with a ligase and a first adaptor molecule, to form a buffer volume comprising a linear DNA product precursor comprising a linear double-stranded region and a first adaptor molecule appended to a first end of the linear region, wherein the first adaptor molecule comprises a first binding moiety;
(b) contacting the buffer volume comprising the linear DNA product precursor with a first substrate under conditions in which the first binding moiety binds to the first substrate in a buffer composition;
(c) washing the first substrate to remove the first buffer volume; and
(d) eluting the bound linear DNA product in a second buffer volume having a different buffer composition, thereby performing a exchanging the buffer composition. The second buffer composition may have a higher or lower salt concentration (different ionic strength) than the first buffer composition. The second buffer composition may have a different salt component than the first buffer composition. The second buffer composition may have a higher or lower pH than the first buffer composition. The first or second buffer may comprise additional components not present in the second or first buffer, respectively, such as EDTA.

### Methods for transcription and protein expression

The invention provides a method for *in vitro* transcription of a purified linear DNA product wherein the method comprises contacting the purified linear DNA product produced by the methods described herein, with a polymerase and producing a transcription product encoded by the linear DNA product.

The invention provides a method for *in vitro* transcription of a linear DNA, wherein the method comprises:
(a) producing a purified linear DNA product by any of the methods described herein;
(b) contacting the purified linear DNA product with a polymerase; and
(c) producing a transcription product encoded by the purified linear DNA product.

The purified linear DNA product may comprise a linear double-stranded region comprising a linear portion of the double-stranded DNA molecule, and wherein the linear double-stranded region is closed at a first end by the first adaptor molecule and closed at a second end by the second adaptor molecule; The method of in vitro transcription may comprise
(a) purifying the closed linear DNA product by any method disclosed herein;
(b) contacting the purified closed linear DNA product with a polymerase; and
(c) producing a transcription product encoded by the closed linear DNA product.

The purified linear DNA product may comprise protected nucleotides, wherein the first and second adaptor molecules are nucleic acid molecules that comprise one or more nuclease-resistant nucleotides (i.e. protected nucleotides); A method for *in vitro* transcription of such a purified linear DNA product may comprise:
(a) purifying the linear double stranded DNA product with protected nucleotides;
(b) contacting the linear DNA product with a polymerase; and
(c) producing a transcription product encoded by the linear DNA product.

The purified linear DNA product may comprise protected nucleotides within the linear DNA product excluding the adaptor molecules (i.e. only within the linear double stranded region), comprises a sense strand and an antisense strand, wherein the linear double-stranded DNA product comprises a single cassette and at least two phosphorothioated nucleotides at internal positions in each strand, wherein the at least two phosphorothioated nucleotides at internal positions in each strand are selected from:
a 5'-end nucleotide of the cassette;
a 3'-end nucleotide of the cassette; and
one or more nucleotides outside of the cassette. A method for *in vitro* transcription of such a purified linear DNA product may comprise:
   (a) purifying the linear double stranded DNA product with phosphorothioated nucleotides;
   (b) contacting the purified linear DNA product with a polymerase; and
   (c) producing a transcription product encoded by the purified linear DNA product.

The linear double stranded product may comprise at least one protected nucleotide. The protected nucleotides may comprise at least 0.01%, at least 0.1% or at least 1% of all nucleotides in the linear DNA product.

The method may use adaptor molecules which comprise both protected nucleotides and adaptor molecules which comprise a hairpin or a stem loop, such as the adaptor molecules described herein, to form a partially closed DNA product wherein the first adaptor molecule is a nucleic acid molecule that comprise one or more nuclease-resistant nucleotides, and wherein the linear double-stranded region is closed at the second end by the second adaptor molecule; A method for *in vitro* transcription of a partially closed linear DNA product may comprise:
(a) purifying the partially closed DNA product;
(b) contacting the partially closed linear DNA product with a polymerase; and
(c) producing a transcription product encoded by the partially closed linear DNA product.

The invention provides a method for producing a protein, wherein the method comprises introducing the linear DNA product purified by the methods described herein, into a cell (e.g. a prokaryotic cell or a eukaryotic cell) or a cell-free expression system to generate a protein encoded by the purified linear DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The purified linear DNA product comprising adaptor molecules or the closed linear DNA product may comprise a cassette. The desired protein may be encoded by the cassette.

The step of introducing the purified linear DNA product into a cell may be performed in vivo or in vitro.

The nuclease-resistant nucleotides (i.e. protected nucleotides) may be any protected nucleotides described herein.

### Methods for cell transfection and cell transfection compositions

The invention provides a method for cell transfection of a purified linear DNA product produced by any of the methods described herein, into a cell.

The invention provides a method for cell transfection of a purified linear DNA product into a cell, wherein the method comprises:
(a) producing a purified linear DNA by any of the methods described herein;
(b) contacting a cell with the purified linear DNA; and
(c) transfecting the purified linear DNA product into the cytosol of the cell.

The invention provides a method for cell transfection of a linear DNA into a cell, wherein the method comprises:
(a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
(b) contacting the linear DNA product precursor with a first substrate under conditions in which the first binding moiety binds to the first substrate;
(c) washing the first substrate to remove unbound linear double-stranded DNA molecules; and
(d) obtaining a purified linear DNA product;
(e) contacting the purified linear DNA product with the cell; and
(f) transfecting the purified linear DNA product into the cytosol of the cell.

The purified linear DNA product may comprise a linear double-stranded region comprising a linear portion of the double-stranded DNA molecule, and wherein the linear double-stranded region is closed at a first end by the first adaptor molecule and closed at a second end by the second adaptor molecule. A method for cell transfection of a closed linear DNA product into a cell may comprise:
(a) purifying the closed linear DNA product by any method disclosed herein;
(b) contacting the closed linear DNA product with the cell; and
(c) transfecting the closed linear DNA product into the cytosol of the cell.

The purified linear DNA product may comprise protected nucleotides and may be purified by a method of the invention, wherein the first and second adaptor molecules are nucleic acid molecules that comprise one or more nuclease-resistant nucleotides (i.e. protected nucleotides). A method for cell transfection of such a purified linear DNA product comprising one or more nuclease-resistant nucleotides may comprise:
(a) purifying the linear double stranded DNA product comprising protected nucleotides;
(b) contacting the purified linear DNA product with the cell; and
(c) transfecting the purified linear DNA product into the cytosol of the cell.

The method may use adaptor molecules which comprise both protected nucleotides and adaptor molecules which comprise a hairpin or a stem loop, such as the adaptor molecules described herein, to form a partially closed DNA product wherein the first adaptor molecule is a nucleic acid molecule that comprise one or more nuclease-resistant nucleotides, and wherein the linear double-stranded region is closed at the second end by the second adaptor molecule and is purified by the methods of the invention. A method for cell transfection of such a purified partially closed linear DNA product may comprise:
(a) purifying the partially closed DNA product;
(b) contacting the purified partially closed linear DNA product with the cell; and
(c) transfecting the purified partially closed linear DNA product into the cytosol of the cell.

The method may use a DNA molecule that comprises internally positioned phosphorothioated nucleotides and is purified by the methods of the invention. A method for cell transfection of such a purified linear DNA product may comprise:
(a) purifying the linear DNA product;
(b) contacting the purified linear DNA product with the cell; and
(c) transfecting the purified linear DNA product into the cytosol of the cell.

The method may use a single stranded DNA product that comprises protected nucleotides and is purified by methods of the invention. A method for cell transfection of such a purified linear DNA product may comprise:
(a) purifying the linear single stranded DNA product;
(b) contacting the purified linear single stranded DNA product with the cell; and
(c) transfecting the purified linear single stranded DNA product into the cytosol of the cell.

The invention provides a cell transfection composition comprising a purified linear DNA product produced by (or obtainable by) the methods described herein.

The cell transfection composition may or may not comprise a carrier e.g. an agent or a formulation. Preferably, the carrier promotes accumulation of the purified linear DNA product at a target site and/or protects the purified linear DNA product from undesirable interactions with biological milieu components and/or protects the purified linear DNA product from metabolism and/or degradation.

The invention further provides a cell transfection method comprising contacting (in vitro) a cell to be transfected with a purified linear DNA product produced by the methods of the invention, and wherein the purified linear DNA product is transfected into the cytosol of the cell.

The cell or cells to be transfected may be provided in a cell culture medium (e.g. in a Petri dish, culture vessel or well, etc). The purified linear DNA product may be added directly to the cell culture medium or the cells may be added to a solution, such as saline, a buffered solution or a cell culture medium, comprising the purified linear DNA product.

The carrier may be a viral carrier or a non-viral carrier. Viral carriers include a lentiviral vector, an adenoviral vector, a retroviral vector, or an adeno associate viral vector for delivery of the linear DNA product. Non-viral carriers (or vectors) include complexing the purified linear DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the purified linear DNA product. The carrier may be a nanoparticulate formulation used to encapsulate the purified linear DNA product.

The carrier may be a modification of the purified linear DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the purified linear DNA product at a target site and/or protects the linear DNA product from metabolism and/or degradation.

The invention further provides a cell obtainable by the methods of the invention. Thus, the cell may comprise a purified linear DNA product produced by the methods of the invention.

The invention further provides a cell transfected with a purified linear DNA product produced by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a purified linear DNA product may be performed in vivo. For example the purified linear DNA product may be administered to an organism (e.g. a subject) in need thereof. The organism (e.g. the subject) may be an animal, such as mammal (e.g. a human), a fungus, a micro-organism, or a plant.

Any or all of the purified linear DNA products described herein may be delivered to a cell via delivery particles such as liposomes, nanoparticles, exosomes, macrovesicles, viral or non-viral vectors. Any or all of the purified linear DNA products described herein may be delivered to a cell using a gene-gun. Any or all of the purified linear DNA products described herein may be delivered to a cell by electroporation. Any or all of the purified linear DNA products described herein may be delivered to a cell by hydrodynamic needle. The purified linear DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a purified DNA product described herein) form an organized structure as a consequence of specific, local interactions among the components themselves, without external direction.

The purified DNA product and the lipid component may reversibly interact to form a self-assembled nanoparticle. The purified DNA product and the lipid component may reversibly interact in the self-assembled nanoparticle through intermolecular forces. The purified DNA product and the lipid component may reversibly interact in the self-assembled nanoparticle through non-covalent interactions. The purified DNA product and the lipid component may reversibly interact in the self-assembled nanoparticle through hydrogen bonds, van der Waals, hydrophobic, and/or electrostatic interactions. The purified DNA product and the lipid component may not be conjugated or linked by forces other than inter-molecular forces in the self-assembled nanoparticle.

### Pharmaceutical compositions and methods for producing pharmaceutical compositions

The invention provides a pharmaceutical composition comprising a purified linear DNA product described herein, and a pharmaceutically acceptable carrier or excipient.

The invention provides a pharmaceutical composition comprising a purified linear DNA product produced by (or obtainable by) the methods described herein, and a pharmaceutically acceptable carrier or excipient.

The invention provides a method for producing a pharmaceutical composition comprising the purified linear DNA product, wherein the method comprises performing the method described herein and formulating the resulting purified linear DNA product with a pharmaceutically acceptable carrier or excipient.

The invention provides a method for producing a pharmaceutical composition, wherein the method comprises:
(a) producing a purified linear DNA product by any of the methods described herein;
(b) formulating the purified linear DNA product with a pharmaceutically acceptable carrier or excipient.

The invention provides a method for producing a pharmaceutical composition, wherein the method comprises:
(a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
(b) contacting the linear DNA product precursor with a first substrate under conditions in which the first binding moiety binds to the first substrate;
(c) washing the first substrate to remove unbound linear double-stranded DNA molecules; and
(d) obtaining a purified linear DNA product.
(e) formulating the purified linear DNA product with a pharmaceutically acceptable carrier or excipient.

The purified linear DNA product may be a linear closed DNA product, and may comprise a linear double-stranded region comprising a linear portion of the double-stranded DNA molecule, and wherein the linear double-stranded region is closed at a first end by the first adaptor molecule and closed at a second end by the second adaptor molecule. A method for producing a pharmaceutical composition may comprise:
(a) purifying the closed linear DNA product; and
(b) formulating the closed linear DNA product with a pharmaceutically acceptable carrier or excipient.

The purified linear DNA product may comprise protected nucleotides, wherein the first and second adaptor molecules are nucleic acid molecules that comprise one or more nuclease-resistant nucleotides (i.e. protected nucleotides). A method for producing a pharmaceutical composition may comprise:
(a) purifying the linear double stranded DNA product with protected nucleotides by any method disclosed herein;
(b) formulating the purified linear DNA product with a pharmaceutically acceptable carrier or excipient.

The purified linear DNA product may comprise protected nucleotides within the purified linear DNA product excluding the adaptor molecules (the protected nucleotides are only within the linear double stranded region), comprises a sense strand and an antisense strand, wherein the purified linear double-stranded DNA product comprises a single cassette and at least two phosphorothioated nucleotides at internal positions in each strand, wherein the at least two phosphorothioated nucleotides at internal positions in each strand are selected from:
i) a 5'-end nucleotide of the cassette;
ii) a 3'-end nucleotide of the cassette; and
iii) one or more nucleotides outside of the cassette.

A method for producing a pharmaceutical composition may comprise:
(a) purifying the linear double stranded DNA product with phosphorothioated nucleotides; and
(b) formulating the purified linear DNA product with phosphorothioated nucleotides with a pharmaceutically acceptable carrier or excipient.

The purified linear double stranded product may comprise at least one protected nucleotide. The protected nucleotides may comprise at least 0.01% preferably at least 0.1%, more preferably at least 1% of all nucleotides in the purified linear DNA product.

The purified linear DNA product may comprise an adaptor molecule which comprise both protected nucleotides and an adaptor molecule which comprises a hairpin or a stem loop, such as the adaptor molecules described herein, to form a partially closed DNA product wherein the first adaptor molecule is a nucleic acid molecule that comprise one or more nuclease-resistant nucleotides, and wherein the linear double-stranded region is closed at the second end by the second adaptor molecule. A method for producing a pharmaceutical composition may comprise:
(a) purifying the partially closed linear DNA product; and
(b) formulating the purified partially closed linear DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the purified linear DNA product can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### Purified Linear DNA products

The invention provides a purified linear DNA product as described herein.

The invention provides a purified closed linear DNA product comprising a linear double-stranded region, wherein the linear double-stranded region comprises a linear portion of a double-stranded DNA molecule, and wherein the linear double-stranded region is closed at a first end by a first adaptor molecule and closed at a second end by the second adaptor molecule.

The invention provides a purified closed linear DNA product comprising a linear portion of a double-stranded DNA molecule, wherein the linear portion of the double-stranded DNA molecule is closed at a first end by a first adaptor molecule and closed at a second end by a second adaptor molecule.

The invention provides a purified linear DNA product comprising a linear double-stranded region, wherein the linear double-stranded region comprises a linear portion of a double-stranded DNA molecule, and wherein the first adaptor molecule is ligated to a first end of the linear double-stranded region and the second adaptor molecule is ligated to a second end of the linear double-stranded region, and wherein the first and second adaptor molecules are nucleic acid molecules that comprise one or more nuclease-resistant nucleotides.

The invention provides a purified linear DNA product comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first and second adaptor molecules are nucleic acid molecules that comprise one or more nuclease-resistant nucleotides.

The invention provides a purified partially closed linear DNA product comprising a linear portion of a double-stranded DNA molecule, wherein a first adaptor molecule is ligated to a first end of the linear portion of the double-stranded DNA molecule and the second adaptor molecule is ligated to a second end of the linear portion of the double-stranded DNA molecule, and wherein the first adaptor molecule is a nucleic acid molecule that comprise one or more nuclease-resistant nucleotides, and wherein the linear portion of the double-stranded DNA molecule is closed at the second end by the second adaptor molecule. Thus, the invention provides a purified partially closed linear DNA product which is closed (or covalently closed) at a second end and open at a first end. The purified partially closed linear DNA product comprises one or more nuclease-resistant nucleotides in the open-end region and/or at the first end. The open-end region may be at the 3' end or 5' end of the molecule.

The open-end region refers to at least 5, at least 10, at least 15, or at least 20 base pairs located closest to the open end of the DNA product. The partially closed linear DNA product may comprise one or more nuclease-resistant nucleotides in a sense and/or an antisense strand. Thus, for example, one or more nucleotides of the 20 base pairs located closest to the open end of the partially closed linear DNA product may be a nuclease-resistant nucleotide. Preferably, the purified partially closed linear DNA product comprises at least 5 nuclease-resistant nucleotides in the open-end region.

The purified partially closed linear DNA product may comprise a double-stranded DNA portion that is closed at a first end and open at a second end. The partially closed linear DNA product may comprise a double-stranded DNA portion that is closed at a first end by a single-stranded portion (i.e. it may comprise a first hairpin at the first end) and open at a second end. The partially closed linear DNA product may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the purified partially closed linear DNA product. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise two or more, three or more, four or more, or five or more nuclease-resistant nucleotides in both the sense and antisense strand. Preferably, the open-end region adjacent to the second end comprises five nuclease-resistant nucleotides in both the sense and antisense strand.

The purified partially closed linear DNA product may comprise a hairpin-loop at the 5' end or the 3' end. The partially closed linear DNA product may comprise a first adaptor molecule at a first end and a second adaptor molecule at a second end. The first adaptor molecule may comprise a hairpin and a second adaptor molecule may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The purified partially closed linear DNA product may be a DNA molecule having a double-stranded portion closed at a first end by ligation of a first adaptor molecule (e.g. hairpin adaptor molecule) to the first end and comprising at a second end a double stranded linear adaptor molecule comprising protected nucleotides (i.e. nucleotides resistant to nuclease (e.g. exonuclease) digestion).

The purified partially closed linear DNA product may comprise (i) a cassette, wherein the cassette comprises a sense strand and an antisense strand; and (ii) one or more nuclease-resistant nucleotides in an open-end region of the purified partially closed linear DNA product, wherein the open-end region is 5' of the sense strand of the cassette. The purified partially closed linear DNA product may comprise (i) a cassette, wherein the cassette comprises a sense strand and an antisense strand; and (ii) one or more nuclease-resistant nucleotides in an open-end region of the purified partially closed linear DNA product, wherein the open-end region is 5' of the antisense strand of the cassette. The purified partially closed linear DNA product may comprise (i) a cassette, wherein the cassette comprises a sense strand and an antisense strand; and (ii) one or more nuclease-resistant nucleotides in an open-end region of the purified partially closed linear DNA product, wherein the open-end region is 3' of the sense strand of the cassette. The purified partially closed linear DNA product may comprise (i) a cassette, wherein the cassette comprises a sense strand and an antisense strand; and (ii) one or more nuclease-resistant nucleotides in an open-end region of the purified partially closed linear DNA product, wherein the open-end region is 3' of the antisense strand of the cassette.

The purified partially closed linear DNA product may comprise (i) a cassette, wherein the cassette comprises a sense strand and an antisense strand; (ii) one or more nuclease-resistant nucleotides in an open-end region of the partially closed linear DNA product, wherein the open-end region is 5' of the sense strand of the cassette; and (iii) one or more nuclease-resistant nucleotides in an open-end region of the partially closed linear DNA product, wherein the open-end region is 3' of the antisense strand of the cassette.

The purified partially closed linear DNA product may comprise (i) a cassette, wherein the cassette comprises a sense strand and an antisense strand; (ii) one or more nuclease-resistant nucleotides in an open-end region of the partially closed linear DNA, wherein the open-end region is 3' of the sense strand of the cassette; and (iii) one or more nuclease-resistant nucleotides in an open-end region of the partially closed linear DNA product, wherein the open-end region is 5' of the antisense strand of the cassette.

The purified closed linear DNA product, linear DNA product or partially closed linear DNA product may comprise a cassette, optionally a single cassette. The linear portion of a double-stranded DNA molecule (of a closed linear DNA product, linear DNA product or partially closed linear DNA product) may comprise a cassette, optionally a single cassette. Accordingly, the cassette (or single cassette) is located between the first and second adaptor molecules.

The term "single cassette" as used herein is intended to encompass a molecules that do not comprise or consist of a plurality of cassettes. That is to say that the closed linear DNA product, linear DNA product, or partially closed linear DNA product, comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The invention provides a purified linear DNA product (e.g. a closed linear DNA product or a partially closed linear DNA product or a single-stranded DNA product or an open ended linear DNA product or a linear DNA product with internally positioned phosphorothioated nucleotides) obtainable by any of the methods described herein.

### Uses and applications

The invention provides a use of a purified linear DNA product as described herein in the production of viral or non-viral delivery system.

The invention provides a use of a purified linear DNA product in the production of viral or non-viral delivery system, wherein the purified linear DNA product is produced by performing the method described herein.

The invention provides a viral or non-viral delivery system comprising a purified linear DNA product as described herein. The invention provides a viral or non-viral delivery system comprising a purified linear DNA product, wherein the purified linear DNA product is produced by performing the method described herein.

### Viral vectors

Methods of producing viral vectors, such as AAV vectors, are known in the art. The most widely used method involves the co-transfection of HEK293 by three bacterial plasmids. The first plasmid encodes the *Rep* and *Cap* element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli*; 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a linear DNA product suitable for use in production of viral vectors. The linear DNA product overcomes the above issues with plasmid vectors.

The invention provides a method of producing a viral vector, the method comprising introducing the purified linear DNA product produced by the methods described herein into a cell under conditions such that the viral vector is produced. The linear DNA product may encode at least one element required for the production of the viral vector. For example, the purified linear DNA product may encode Rep and/or Cap elements. The purified linear DNA product may encode the helper plasmid elements. The purified linear DNA product may encode Rep, Cap and helper plasmid elements. The purified linear DNA product may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the purified linear DNA product produced by the methods described herein is suitable for use in production of non-viral vectors. The purified linear DNA product produced by the methods of the invention overcomes the issues of using plasmid vectors in non-viral vector preparation. For example, the purified linear DNA product, unlike plasmid DNA, does not comprise a bacterial backbone, allowing more transgene copies per mg of DNA. In addition, the purified linear DNA product does not comprise antibiotic resistant genes and bacterial contaminants. Moreover, the purified linear DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the purified linear DNA product with the cell. The cell may be an animal cell, preferably mammal cell, such as human cell.

The invention also provides a cell obtainable by the methods described herein.

### General therapeutic and diagnostic uses

The purified linear DNA product produced by the methods described herein is particularly suitable for use in therapy. The invention provides a purified linear DNA product as described herein for use in therapy. The invention provides a purified linear DNA product obtainable by the method described herein for use in therapy. The purified linear DNA product may encode a sequence of a therapeutic protein, a part of a vaccine, or an element of a genetic engineering mechanism, which is used to treat a disease or infection in a subject.

The invention further provides the purified linear DNA product as described herein for use as a medicament. The invention further provides the purified linear DNA product obtainable by the methods described herein for use as a medicament. The invention also provides the use of a purified linear DNA product as described herein for the manufacture of a medicament for treating a disease. The invention also provides the use of a purified linear DNA product obtainable by the methods described herein for the manufacture of a medicament for treating a disease.

The purified linear DNA product may encode a sequence of a therapeutic protein, a part of a vaccine, or an element of a genetic engineering mechanism, which is used to treat a disease or infection in a subject.

The invention further provides the purified linear DNA product produced by the methods described herein for use in treating a disease.

The invention also provides a method of treating a disease in a subject comprising administering to the subject a purified linear DNA product described herein. The invention also provides a method of treating a disease in a subject comprising administering to the subject the purified linear DNA product obtainable by the methods described herein. Preferably, the amount of the purified linear DNA product administered to the subject is a therapeutic active amount.

The purified linear DNA product described herein may be used to treat any disease or disorder. For example, the purified linear DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the purified linear DNA product is used to treat a genetic disorder. More preferably still, the linear DNA product is used to treat a monogenic disorder. For example, the purified linear DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the purified linear DNA product may receive the purified linear DNA product in the form of any of the pharmaceutical compositions described herein.

A subject treated with the purified linear DNA product may receive the purified linear DNA product in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the purified linear DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the linear DNA product that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the purified linear DNA product as described herein in a method of diagnosing a disease and/or a disorder. The invention also provides the use of the purified linear DNA product obtainable by the methods described herein in a method of diagnosing a disease and/or a disorder.

The invention provides the use of the purified linear DNA product in the "in vitro" diagnosis of a disease. The invention provides the use of the purified linear DNA product obtainable by the methods described herein in the "in vitro" diagnosis of a disease.

The invention also provides the purified linear DNA product for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the purified linear DNA product is used to diagnose a genetic disorder. More preferably still, the purified linear DNA product is used to diagnose a monogenic disorder. For example, the purified linear DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the purified linear DNA product.

To facilitate detection and/or quantification of the purified linear DNA product, the purified linear DNA product may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the purified linear DNA product. For example, the signal may be measured by flow cytometry and/or fluorescence-activated cell sorting of a purified linear DNA product attached to a fluorescent probe.

The purified linear DNA product may be detected by being bound to a capture moiety, for example in a lateral flow assay. In this example, the functional portion is a protein, for example, an antibody specific for the capture moiety. The capture of the antibody attached to the purified linear DNA product may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the purified linear DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the purified linear DNA product described herein for use in cell therapy. The invention provides the purified linear DNA product obtainable by the methods described herein for use in cell therapy.

The invention provides the purified linear DNA product described herein for use in cell therapy. The invention provides the purified linear DNA product obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

### Vaccines

The purified linear DNA products produced by the methods described herein are particularly suitable for use in vaccine production. A vaccine may comprise a purified linear DNA product described herein. A vaccine may comprise a purified linear DNA product obtainable by the methods described herein. Alternatively, the purified linear DNA product may be used to produce a vaccine, preferably an mRNA-based vaccine. For example, vaccines such as the BioNTech and Moderna mRNA vaccines against COVID-19.

Thus, the invention provides the use of the purified linear DNA product described herein in the production of a vaccine. The invention also provides the use of the purified linear DNA product obtainable by the methods described herein in the production of a vaccine.

The purified linear DNA product may encode an antigen, which may cause an immune response in a subject. The subject may be human. Preferably, the antigen is encoded on the cassette.

### CAR-T cells

The invention provides the use of a purified linear DNA product described herein in the production of a CAR-T cell. The invention provides the use of the purified linear DNA product obtainable by the methods described herein in the production of a CAR-T cell.

The invention provides a method for producing a genetically engineered CAR-T cell comprising: (a) introducing the purified linear DNA product described herein into a T cell; and (b) expressing a gene of interest encoding by the purified linear DNA product. Preferably, the gene of interest is a tumour-specific CAR.

The invention provides a method for producing a genetically engineered CAR-T cell comprising: (a) introducing the purified linear DNA product obtainable by the methods described herein into a T cell; and (b) expressing a gene of interest encoding by the purified linear DNA product. Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

The invention also provides an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The purified linear DNA product may comprise a gene sequence encoding any component of the CRIPSR machinery. The purified linear DNA product may encode all components of the CRIPSR machinery.

The purified linear DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The linear DNA product encoding the repair template may be delivered to a cell by a nanoparticle, a non-viral vector or a viral vector, or without the aid of any carrier. The purified linear DNA product encoding the repair template may be delivered to a cell by electroporation. The purified linear DNA product encoding the repair template may be delivered to a cell by hydrodynamic needle.

The purified linear DNA product may comprise (or further comprise) a gene sequence encoding a nuclease protein of a CRISPR system (e.g. Cas9, Cpf1, or MAD7), and/or a guide RNA. The purified linear DNA product may comprise (or further comprise) a gene sequence encoding a nuclease protein of a CRISPR system (e.g. Cas9, Cpf1, or MAD7). The purified linear DNA product may comprise (or further comprise) a gene sequence encoding a guide RNA. The purified linear DNA product may comprise (or further comprise) a gene sequence encoding a nuclease protein of a CRISPR system (e.g. Cas9, Cpf1, or MAD7) and a guide RNA. The purified linear DNA product may comprise (or further comprise) a gene sequence encoding a genomic target to be modified (e.g. a spacer). The purified linear DNA product may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the linear DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The purified linear DNA product may encode the nuclease of the CRISPR system and guide RNA. One linear purified DNA product may encode the nuclease of the CRISPR system, and the other purified linear DNA product may encode guide RNA.

The purified linear DNA product may be for use in CRISPR-Cas mediated repair by recombination, homology-directed repair, or non-homologous end joining.

If the nuclease of the CRISPR system and the guide RNA are encoded by a different purified linear DNA product, they may be part of a different or the same delivery mechanism. For example, the purified linear DNA product encoding the nuclease of the CRISPR system may be delivered to a cell by a first nanoparticle, non-viral vector or viral vector, whereas the purified linear DNA product encoding the guide RNA may be delivered to a cell by a second nanoparticle, a non-viral vector, or viral vector. For example, the purified linear DNA product encoding the nuclease of the CRISPR system and the purified linear DNA product encoding the guide RNA may be delivered to a cell by the same nanoparticle, non-viral vector or viral vector.

If the nuclease of the CRISPR system and the guide RNA are encoded by the same purified linear DNA product (or by a vector that comprises the purified linear DNA product) they may be part of the same delivery mechanism. For example, the purified linear DNA product, or the vector, encoding the nuclease of the CRISPR system and the guide RNA may be delivered to a cell by a nanoparticle, a non-viral vector or a viral vector.

The purified linear DNA product encoding the nuclease of the CRISPR system and the purified linear DNA product encoding the guide RNA may be delivered to a cell by electroporation. The purified linear DNA product encoding the nuclease of the CRISPR system and the purified linear DNA product encoding the guide RNA may be delivered to a cell by hydrodynamic needle.

Thus, the invention provides the purified linear DNA product described herein for use in CRISPR system delivery to a cell. The invention provides a method of delivering the CRISPR system to a cell, comprising contacting the purified linear DNA product described herein with a cell. The invention also provides the purified linear DNA product obtainable by the methods described herein for use in CRISPR system delivery to a cell. The invention provides a method of delivering the CRISPR system to a cell, comprising contacting the purified linear DNA product obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The purified linear DNA product produced by the methods described herein may be used in transcription, to generate RNA, preferably mRNA, in vitro or in vivo.

### Kits

The invention provides a kit comprising components required to carry out the method described herein. The kit comprises at least:
(a) first adaptor molecule comprising a first binding moiety;
(b) a ligase; and
(c) a substrate to which the first binding moiety binds.

The kit may additionally comprise a second adaptor molecule (optionally comprising a second binding moiety), a second substrate, at least one buffer and/or a nuclease.

The first adaptor molecule and/or the second adaptor molecule may comprise any one of the sequences of SEQ ID NO: 1, 2, 3, 4, 5, 6, 7, 8, 9, 10 a portion thereof. The first adaptor molecule and/or the second adaptor molecule may comprise at least 13, at least 14, at least 15, at least 16, at least 17, at least 18, or at least 19 contiguous nucleotides of SEQ ID NO: 1, 2, 3,4, 5, 6, 7, 8, 9, or 10. The first and second adaptor molecules may comprise an identical nucleic acid sequence. The first and second adaptor molecules may comprise a different nucleic acid sequence. The first adaptor molecule may comprise one or more protected nucleotides and the second adaptor molecule may comprise a hairpin or a stem loop region.

The first and second adaptor molecule may be provided in a kit together or separately.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The endonuclease may be a restriction enzyme endonuclease. The endonuclease may or may not be a Type IIS restriction enzyme. The endonuclease may or may not be any enzyme that recognizes a DNA sequence and cleaves outside of the recognition sequence. For example, the endonuclease may or may not be a Bbsl, Bsal, BsmBI, BspQI, BtgZI, Esp3I,SapI, Aarl, Acc36I, AclWI, Acul, Ajul, AloI, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BslFI, BsmAI, BsmFI, Bsml, Bso31I, BspCNI, BspMI, BspPI, BspQI, BspTNI, BsrDI, Bsrl, Bst6I, BstF5I, BstMAI, BstV1I, BstV2I, Bsul, BtgZI, BtsCI, Btsl-v2, BtsMutl, Bvel, Csel, CspCI, Eam11041, Earl, Ecil, Eco31I, Eco57I, Esp3I, Faql, Faul, Fokl, Gsul, Hgal, Hphl, HpyAV, Lgul, Lmnl, Lsp1109I, LweI, Mboll, Mlyl, Mmel, Mnll, Mva1269I, NmeAIII, PaqCI, PciSI, PctI, Plel, Ppsl, Psrl, Schl, SfaNI, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or *E. coli* DNA ligase.

The nuclease may be an exonuclease e.g. exonuclease I, exonuclease III and/or exonuclease VIII.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

| **SEQ ID NO:** | **Sequence 5' to 3'** |
|---|---|
| SEQ ID NO:1 | AGGGCTAACATTTGTTGGCC |
| SEQ ID NO:2 | AGGGCTAACATTTGTTGGCC |
| SEQ ID NO:3 | AGGGCTAACATTTGTTGGCC |
| SEQ ID NO:4 | G*G*C*C*A*ACAAATGTTAG |
| SEQ ID NO:5 | TTTTTTTTTTTTTT |
| SEQ ID NO:6 | TTTTTTTTTTTTTT |
| SEQ ID NO:7 | TTTTTTTTTTTTTT |
| SEQ ID NO:8 | AAAAAAAAAAAAA*A*A*A*A*A |
| SEQ ID NO:9 | AGGGAGTCTCCTTGATGGCCTCTCTGGCCATCAAGGAGACT |
| SEQ ID NO:10 | AGGGAGTCTCCTTGTTGGCCACTCAGGCCAACAAGGAGACT |

| | |
|---|---|
| Table of sequences disclosed in the application * indicates a phosphorothioate linkage, and underlined bases (e.g., T) indicates a biotinylated base. | |

When the linear DNA to be purified is a linear DNA product comprising internally positioned protected nucleotides, the first and/or second adaptor molecule may comprise:
SEQ ID NO:4 and SEQ ID NO:1;
SEQ ID NO:4 and SEQ ID NO:2;
SEQ ID NO:4 and SEQ ID NO:3;
SEQ ID NO:8 and SEQ ID NO:5;
SEQ ID NO:8 and SEQ ID NO:6;
SEQ ID NO:8 and SEQ ID NO:7;
SEQ ID NO:9; and/or
SEQ ID NO:10.

For an open ended linear DNA product precursor or a purified open ended linear DNA product, the first and/or second adaptor molecule may comprise:
SEQ ID NO:4 and SEQ ID NO:1;
SEQ ID NO:4 and SEQ ID NO:2; and/or
SEQ ID NO:4 and SEQ ID NO:3;

For partially closed linear DNA product precursor or a purified partially closed liner DNA product, the first or second adaptor molecule may comprise:
SEQ ID NO:8 and SEQ ID NO:5;
SEQ ID NO:8 and SEQ ID NO:6; or
SEQ ID NO:8 and SEQ ID NO:7;

And the other adaptor molecule may comprise:
SEQ ID NO:9 or SEQ ID NO:10.

For a closed linear DNA product precursor or a purified closed linear DNA product, the first and/or second adaptor molecule may comprise SEQ ID NO:9 or SEQ ID NO:10

### Clauses

1. A method for purification of a linear DNA product wherein the method comprises:
   (a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
   (b) contacting the linear DNA product precursor with a first substrate under conditions in which the first binding moiety binds to the first substrate;
   (c) washing the first substrate to remove unbound linear double-stranded DNA molecules; and
   (d) obtaining a purified linear DNA product.
2. The method of clause 1, wherein the method further comprises appending a second adaptor molecule to a second end of the linear double-stranded region.
3. The method of clause 2, wherein the second adaptor molecule comprises a second binding moiety.
4. The method of any one of clauses 1 to 3, wherein the first and/or second adaptor molecule is a DNA adaptor molecule.
5. The method of any one of clauses 2 to 4, wherein the first and second adaptor molecule are different.
6. The method of any one of clauses 2 to 5, wherein the first and second binding moiety are different.
7. The method of any one of clauses 2 to 6, further comprising contacting the linear DNA product with a second substrate under conditions in which the second binding moiety binds to the second substrate.
8. The method of any one of clauses 1 to 7, wherein the first and/or second adaptor molecule has a double stranded region and an overhang.
9. The method of any one of clauses 1 to 8, wherein the first and/or the second adaptor molecule has a double stranded region and a blunt end.
10. The method of any one of clauses 1 to 9, wherein the first and/or the second adaptor molecule comprises double stranded region and a hairpin.
11. The method of any one of clauses 1 to 11, wherein the linear double-stranded region comprises a first strand and a second strand, and the linear DNA product precursor does not comprise a binding moiety on the first strand
12. The method of any one of clauses 1 to 11, wherein the linear double-stranded region comprises a first strand and an second strand, and the linear DNA product precursor does not comprise a binding moiety on the second strand
13. The method of clause any one of clauses 11 or 12, wherein linear DNA product is single stranded and the step of obtaining the purified linear DNA product is performed under denaturing conditions.
14. The method of any one of clauses 11 or 12, wherein the linear DNA product is single stranded and contacting the linear DNA product precursor with a first and/or second substrate is performed under denaturing conditions.
15. The method of any one of clauses 1 to 14, wherein the first adaptor molecule and/or second adaptor molecule comprises a cleavable endonuclease target sequence between the cassette and the binding moiety.
16. The method of clause 15, wherein the step of obtaining the purified linear DNA product is performed by releasing the linear DNA product from the substrate by endonuclease digestion with a restriction endonuclease having a target sequence in the first and/or second adaptor molecule.
17. The method of any one of clauses 1 to 15, wherein the step of obtaining the purified linear DNA product is performed under eluting conditions.
18. The method of any one of clauses 2 to 18, wherein contacting the linear DNA product with the first substrate and the second substrate is carried out simultaneously or sequentially.
19. The method of any one of clauses 1 to 18, wherein the binding of the first or second moiety to the first or second substrate is by non-covalent bonds, such as hydrogen bonds, van de Waals forces, hydrophobic bonds or ionic bonds.
20. The method of any one of clauses 1 to 19, wherein the linear region comprises a cassette.
21. The method of any one of clauses 1 to 20, wherein the linear DNA product is resistant to exonuclease digestion.
22. The method of any one of clauses 20 to 21, wherein the linear DNA product is double stranded and comprises at least two phosphorothioated nucleotides at internal positions in each strand, wherein the at least two phosphorothioated nucleotides at internal positions in each strand are selected from:
   (a) a 5'-end nucleotide of the cassette;
   (b) a 3'-end nucleotide of the cassette; and
   (c) one or more nucleotides outside of the cassette.
23. The method of any one of clauses 20 to 22, wherein linear DNA product is double stranded and the cassette comprises a coding sequence comprising a sense strand and an antisense strand, and wherein in the sense strand:
   (a) one of the at least two phosphorothioated nucleotides is the 5'-end nucleotide of the cassette; and/or one of the at least two phosphorothioated nucleotides is in a first region of the sense strand, wherein the first region of the sense strand is 5' of the cassette; and
   (b) one of the at least two phosphorothioated nucleotides is the 3'-end nucleotide of the cassette; and/or one of the at least two phosphorothioated nucleotides is in a second region of the sense strand, wherein the second region of the sense strand is 3' of the cassette.
24. The method of any one of clauses 1 to 23, wherein the first adaptor molecule and/or the second adaptor molecule is a synthetic adaptor molecule.
25. The method of any one of clauses 1 to 24, wherein the first adaptor molecule and/or the second adaptor molecule comprise a single-stranded portion, optionally wherein:
   (a) the single-stranded portion forms a hairpin;
   (b) the single-stranded portion comprises less than 10, 9, 8, 7, 6, 5, 4, 3, 2 nucleotides; and/or
   (c) the single-stranded portion comprises 5 nucleotides.
26. The method of any one of clauses 1 to 25, wherein the first adaptor molecule and/or the second adaptor molecule comprises a double stranded portion, optionally wherein:
   (a) the double-stranded portion comprises less than 50, 45, 40, 35, 30 base pairs; and/or
   (b) the double-stranded portion comprises at least 10, 11, 12, 13, 14, 15 base pairs.
27. The method of any one of clauses 2 to 26, wherein the linear double-stranded region is closed at a first end by the first adaptor molecule and closed at a second end by the second adaptor molecule.
28. The method of any one of clauses 2 to 27, wherein the first adaptor molecule comprises one or more nuclease-resistant nucleotides (i.e. protected nucleotides) and wherein the linear double-stranded region is closed at the second end by the second adaptor molecule.
29. The method of any one of clauses 2 to 28, wherein the first and second adaptor molecules each comprise one or more nuclease-resistant nucleotides (i.e. protected nucleotides).
30. The method of any one of clauses 1 to 29 wherein the first adaptor molecule and/or the second adaptor molecule comprises at least one biotinylated nucleotide and the first and/or second substrate comprises a biotin-binding substrate, such as streptavidin or an anti-biotin antibody.
31. The method of any one of clauses 1 to 30, wherein the first adaptor molecule and/or the second adaptor molecule comprises an aptamer and the first and/or second substrate comprises the aptamer target molecule specific to the aptamer.
32. The method of clause 31, wherein the first adaptor molecule and the second adaptor molecule comprise different aptamers and the first and second substrate comprise different aptamer target molecules.
33. The method of any one of clauses 1 to 32 wherein the first adaptor molecule and/or the second adaptor molecule comprises a homopolymeric sequence, such as polyA, polyT, polyG or polyC and the first and/or second substrate comprises a complementary oligonucleotide sequence.
34. The method of any one of clauses 1 to 33, wherein the first adaptor molecule and/or the second adaptor molecule comprises an antibody or antibody binding fragment and the first and/or second substrate comprises the antibody antigen.
35. The method of clause 34, wherein the first adaptor molecule and the second adaptor molecule comprise different antibodies or antibody binding fragments and the first and second substrate comprise different antigens.
36. The method of any one of clauses 1 to 35 x, wherein the first adaptor molecule and/or the second adaptor molecule comprises an antigen and the first and/or second substrate comprises an antibody or antibody binding fragment that binds to the antigen.
37. The method of clause 36, wherein the first adaptor molecule and the second adaptor molecule comprise different antigens and the first and second substrate comprise different antibodies or antibody binding fragments that bind to the respective antigens.
38. The method of any one of clauses 1 to 37, wherein the first adaptor molecule and/or the second adaptor molecule comprises at least one phosphorothioated nucleotide.
39. The method of any one of clauses 1 to 18 wherein the linear DNA product comprises at least 0.01% phosphorothioated nucleotides, optionally at least 0.1% or optionally at least 1% phosphorothioated nucleotides.
40. The method of any one of clauses 1 to 39 wherein the first adaptor molecule and/or the second adaptor molecule comprises a hairpin loop/ ligation of the first or second adaptor molecule results in a closed first and/or second end of the DNA product.
41. The method of clause 40, wherein the hairpin loop comprises a biotinylated nucleotide.
42. The method of any one of clauses 1 to 41, wherein the first and/or second adaptor molecule comprises a biotinylated nucleotide, an aptamer or a homopolymeric sequence.
43. The method of any one of clauses 1 to 42, wherein the first and/or second adaptor molecule comprises an antibody or antibody binding fragment, a His Tag, a GST tag.
44. The method of clause 43 wherein the antibody or antibody binding fragment, a His Tag, a GST tag is attached to the first and or second adaptor molecule via a linker or a spacer.
45. The method of any one of clauses 1 to 44, further comprising contacting the linear DNA product precursor with an exonuclease prior to being contacted with the first and/or second substrate.
46. The method of any one of clauses 1 to 46, wherein the method comprises, prior to contacting the double-stranded DNA molecule with a ligase and a first adaptor molecule, the step of rolling circle amplification of a DNA template molecule comprising at least one endonuclease target sequence to generate a double-stranded DNA molecule.
47. The method of clause 46, wherein the method further comprises contacting the double stranded DNA molecule with an endonuclease having the at least one target sequence prior to or simultaneously with contacting the double stranded DNA molecule with a ligase and a first adaptor molecule.
48. A method for concentration of a linear DNA product comprising the steps of:
   (a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
   (b) contacting the linear DNA product precursor with a first substrate under conditions in which the first binding moiety binds to the first substrate in a buffer volume;
   (c) eluting the bound linear DNA product in a lower buffer volume; and
   (d) obtaining a concentrated linear DNA product.
49. A method of exchanging a buffer composition of a buffer volume comprising a linear DNA product, wherein the method comprises the steps of:
   (a) contacting the buffer volume comprising a linear double-stranded DNA molecule with a ligase and a first adaptor molecule, to form a buffer volume comprising a linear DNA product precursor comprising a linear double-stranded region and a first adaptor molecule appended to a first end of the linear region, wherein the first adaptor molecule comprises a first binding moiety;
   (b) contacting the buffer volume comprising the linear DNA product precursor with a first substrate under conditions in which the first binding moiety binds to the first substrate in a buffer composition;
   (c) washing the first substrate to remove the first buffer volume; and
   (d) eluting the bound linear DNA product in a second buffer volume having a different buffer composition, thereby performing a exchanging the buffer composition.
50. A linear DNA product precursor or a purified linear DNA product comprising a double stranded linear region, and a first adaptor molecule appended to a first end of the product precursor, wherein the first adaptor molecule comprises a first binding moiety.
51. The linear DNA product precursor or a purified linear DNA product of clause 50, further comprising a second adaptor molecule appended to a second end of the linear region.
52. The linear DNA product precursor or a purified linear DNA product of clause 51, wherein the second adaptor molecule comprises a second binding moiety.
53. The linear DNA product precursor or a purified linear DNA product of any one of clauses 50 to 52, wherein the first and/or second adaptor molecule is a DNA adaptor molecule.
54. The linear DNA product precursor or a purified linear DNA product of any one of clauses 51 to 53, wherein the first and second adaptor molecule are different.
55. The linear DNA product precursor or a purified linear DNA product of any one of clauses 52 to 54, wherein the first and second binding moiety are different.
56. The linear DNA product precursor or a purified linear DNA product of any one of clauses 52 to 55, further comprising contacting the linear DNA product with a second substrate under conditions in which the second binding moiety binds to the second substrate.
57. The linear DNA product precursor or a purified linear DNA product of any one of clauses 50 to 56, wherein the first and/or second adaptor molecule has a double stranded region and an overhang.
58. The linear DNA product precursor or a purified linear DNA product of any one of clauses 50 to 57, wherein the first and/or the second adaptor molecule has a double stranded region and a blunt end.
59. The linear DNA product precursor or a purified linear DNA product of any one of clauses 50 to 58, wherein the first and/or the second adaptor molecule comprises double stranded region and a hairpin.
60. The linear DNA product precursor or a purified linear DNA product of any one of clauses 50 to 59, wherein the linear double-stranded region comprises a first strand and a second strand, and the linear DNA product precursor does not comprise a binding moiety on the first strand
61. The linear DNA product precursor or a purified linear DNA product of any one of clauses 50 to 60, wherein the linear double-stranded region comprises a first strand and an second strand, and the linear DNA product precursor does not comprise a binding moiety on the second strand
62. The linear DNA product precursor or a purified linear DNA product of any one of clauses 50 to 61, wherein the first adaptor molecule and/or second adaptor molecule comprises an endonuclease target sequence between the linear double stranded region and the binding moiety.
63. The linear DNA product precursor or a purified linear DNA product of any one of clauses 50 to 62, wherein the linear region comprises a cassette.
64. The linear DNA product precursor or a purified linear DNA product of any one of clauses 50 to 63, wherein the linear DNA product is resistant to exonuclease digestion.
65. The linear DNA product precursor or a purified linear DNA product of any one of clauses 63 to 64, wherein the linear DNA product is double stranded and comprises at least two phosphorothioated nucleotides at internal positions in each strand, wherein the at least two phosphorothioated nucleotides at internal positions in each strand are selected from:
   (a) a 5'-end nucleotide of the cassette;
   (b)a 3'-end nucleotide of the cassette; and
   (c) one or more nucleotides outside of the cassette.
66. The linear DNA product precursor or a purified linear DNA product of any one of clauses 63 to 65, wherein linear DNA product or precursor is double stranded and the cassette comprises a coding sequence comprising a sense strand and an antisense strand, and wherein in the sense strand:
   (a) one of the at least two phosphorothioated nucleotides is the 5'-end nucleotide of the cassette; and/or one of the at least two phosphorothioated nucleotides is in a first region of the sense strand, wherein the first region of the sense strand is 5' of the cassette; and
   (b) one of the at least two phosphorothioated nucleotides is the 3'-end nucleotide of the cassette; and/or one of the at least two phosphorothioated nucleotides is in a second region of the sense strand, wherein the second region of the sense strand is 3' of the cassette.
67. The linear DNA product precursor or a purified linear DNA product of any one of clauses 50 to 66, wherein the first adaptor molecule and/or the second adaptor molecule is a synthetic adaptor molecule.
68. The linear DNA product precursor or a purified linear DNA product of any one of clauses 50 to 67, wherein the first adaptor molecule and/or the second adaptor molecule comprise a single-stranded portion, optionally wherein:
   (a) the single-stranded portion forms a hairpin;
   (b) the single-stranded portion comprises less than 10, 9, 8, 7, 6, 5, 4, 3, 2 nucleotides; and/or
   (c) the single-stranded portion comprises 5 nucleotides.
69. The linear DNA product precursor or a purified linear DNA product of any one of clauses 50 to 68, wherein the first adaptor molecule and/or the second adaptor molecule comprises a double stranded portion, optionally wherein:
   (a) the double-stranded portion comprises less than 50, 45, 40, 35, 30 base pairs; and/or
   (b) the double-stranded portion comprises at least 10, 11, 12, 13, 14, 15 base pairs.
70. The linear DNA product precursor or a purified linear DNA product of any one of clauses 50 to 69, wherein the linear double-stranded region is closed at a first end by the first adaptor molecule and closed at a second end by the second adaptor molecule.
71. The linear DNA product precursor or a purified linear DNA product of any one of clauses 50 to 70, wherein the first adaptor molecule comprises one or more nuclease-resistant nucleotides (i.e. protected nucleotides) and wherein the linear double-stranded region is closed at the second end by the second adaptor molecule.
72. The linear DNA product precursor or a purified linear DNA product of any one of clauses 51 to71, wherein the first and second adaptor molecules each comprise one or more nuclease-resistant nucleotides (i.e. protected nucleotides).
73. The linear DNA product precursor or a purified linear DNA product of any one of clauses 50 to 72 wherein the first adaptor molecule and/or the second adaptor molecule comprises at least one biotinylated nucleotide.
74. The linear DNA product precursor or a purified linear DNA product of any one of clauses 50 to 73, wherein the first adaptor molecule and/or the second adaptor molecule comprises an aptamer.
75. The linear DNA product precursor or a purified linear DNA product of any one of clauses 51 to 74, wherein the first adaptor molecule and the second adaptor molecule comprise different aptamers.
76. The linear DNA product precursor or a purified linear DNA product of any one of clauses 50 to 75 wherein the first adaptor molecule and/or the second adaptor molecule comprises a homopolymeric sequence, such as polyA, polyT, polyG or polyC.
77. The linear DNA product precursor or a purified linear DNA product of any one of clauses 50 to 76, wherein the first adaptor molecule and/or the second adaptor molecule comprises an antibody or antibody binding fragment.
78. The linear DNA product precursor or a purified linear DNA product of any one of clauses 51 to 77, wherein the first adaptor molecule and the second adaptor molecule comprise different antibodies or antibody binding fragments..
79. The linear DNA product precursor of clause x, wherein the first adaptor molecule and/or the second adaptor molecule comprises an antigen.
80. The linear DNA product precursor or a purified linear DNA product of any one of clauses 51 to 79, wherein the first adaptor molecule and the second adaptor molecule comprise different antigens.
81. The linear DNA product precursor or a purified linear DNA product of any one of clauses 50 to 80 wherein the first adaptor molecule and/or the second adaptor molecule comprises at least one phosphorothioated nucleotide.
82. The linear DNA product precursor or a purified linear DNA product of clause 50 to 81 wherein the linear DNA product comprises at least 0.01% phosphorothioated nucleotides, optionally at least 0.1% or optionally at least 1% phosphorothioated nucleotides.
83. The linear DNA product precursor or a purified linear DNA product of any one of clauses 50 to 82 wherein the first adaptor molecule and/or the second adaptor molecule comprises a hairpin loop
84. The linear DNA product precursor or a purified linear DNA product of clause 83, wherein the hairpin loop comprises a biotinylated nucleotide.
85. The linear DNA product precursor or a purified linear DNA product of any one of clauses 50 to 84, wherein the first and/or second adaptor molecule comprises a biotinylated nucleotide, an aptamer or a homopolymeric sequence.
86. The linear DNA product precursor or a purified linear DNA product of any one of clauses 50 to 85, wherein the first and/or second adaptor molecule comprises an antibody or antibody binding fragment, a His Tag, a GST tag.
87. The linear DNA product precursor or a purified linear DNA product of clause 86 wherein the antibody or antibody binding fragment, a His Tag, a GST tag is attached to the first and/or second adaptor molecule via a linker or a spacer.
88. A purified linear DNA product obtainable by a method of any one of clauses 1 to 49.
89. A method for in vitro transcription of a purified linear DNA product, the method comprising the steps of:
   (a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
   (b) contacting the linear DNA product precursor with a first substrate under conditions in which the first binding moiety binds to the first substrate;
   (c) washing the first substrate to remove unbound linear double-stranded DNA molecules;
   (d) obtaining a purified linear DNA product;
   (e) contacting the purified linear DNA product with a polymerase; and
   (f) producing a transcription product.
90. A method for protein expression, the method comprising the steps of:
   (a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
   (b) contacting the linear DNA product precursor with a first substrate under conditions in which the first binding moiety binds to the first substrate;
   (c) washing the first substrate to remove unbound linear double-stranded DNA molecules;
   (d) obtaining a purified linear DNA product;
   (e) introducing the purified linear DNA product into a prokaryotic cell or a eukaryotic cell or a cell-free protein expression system to generate a desired RNA or protein.
91. A method for cell transfection of a purified linear DNA product into a cell, the method comprising the steps of:
   (a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
   (b) contacting the linear DNA product precursor with a first substrate under conditions in which the first binding moiety binds to the first substrate;
   (c) washing the first substrate to remove unbound linear double-stranded DNA molecules;
   (d) obtaining a purified linear DNA product;
   (e) contacting the purified linear DNA product with the cell; and
   (f) transfecting the purified linear DNA product into the cytosol of the cell.
92. A method for producing a pharmaceutical composition comprising a purified linear DNA product, the method comprising performing the method of any one of clauses 1 to 49 and formulating the resulting purified linear DNA product with a pharmaceutically acceptable carrier or excipient.
93. A purified linear DNA product according to any one of clauses 50 to 88 for use in therapy.
94. Use of a purified linear DNA product in the production of a viral or non-viral delivery system, wherein the purified linear DNA product is purified by performing the method of any one of clauses 1 to 49.
95. Use of a purified linear DNA product in the manufacture of a medicament for treatment of a human or animal body by therapy, wherein the manufacture comprises performing the method of any one of clauses 1 to 49
96. A kit comprising:
   (a) a first adaptor molecule comprising a first binding moiety;
   (b) a ligase; and
   (c) a substrate to which the first binding moiety binds.
97. A kit of clause 96, further comprising a second adaptor molecule.
98. A kit of clause 97, wherein the second adaptor molecule comprises a second binding moiety.

### EXAMPLES

### Example 1: Rolling circle amplification, digestion and appending adaptor molecules to resulting double-stranded DNA molecule

Cre recombinase from the P1 bacteriophage is a Type I topoisomerase. The enzyme catalyses the site-specific recombination of DNA between IoxP sites. LoxP recognition site (34 bp) consists of two 13 bp inverted repeats which flank an 8 bp spacer region, which confers directionality. The products of Cre-mediated recombination are dependent upon the location and relative orientation of the IoxP sites. Two DNA species containing single IoxP sites were fused. DNA found between two IoxP sites oriented in the same direction was excised as a circular loop of DNA, while DNA between opposing IoxP sites was inverted with respect to external sequences. Cre recombinase requires no additional cofactors or accessory proteins for its function.

Cre reaction conditions: reaction volume 50 µl, DNA of interest purified from agarose gel electrophoresis after restriction enzyme digestion (100 ng), Cre recombinase (NEB, 4 units), incubation time and temperature: 30 min at 37°C and 20 min at 80°C. Next, to remove remaining noncircular DNA molecules before the amplification step, *E. coli* exonuclease I (NEB, 20 units) and III (NEB, 100 units) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like *Tth*PrimPol (Picher *et al,* 2016) that synthesizes the primers for Phi29DNApol during the amplification reaction.

Before the amplification, circularized DNA samples were first denatured by adding 1 volume of buffer D (400 mM KOH, 10 mM EDTA) and incubating 3 min at room temperature. Samples were then neutralized by adding 1 volume of buffer N (400 mM HCI, 600 mM Tris-HCI pH 7.5). Rolling circle amplification conditions: 10 ml reaction volume, 1 ml TruePrime WGA reaction buffer 10x (4basebio), 500 µl denatured DNA sample, 1 ml *Tth*PrimPol (1 µM), 160 µl QualiPhi Phi29DNApol (12,5 µM), 2.5 units PPase (Thermo) and 1 ml dNTPs (10 mM). Incubation time and temperature: 20 hours at 30°C and 10 min at 65°C.

Amplified DNAs were incubated with Type II restriction enzyme Bsal, T4 DNA ligase and complementary adaptor molecules to the 5' protruding ends generated by Bsal on the amplified DNA. Digestion and ligation reaction conditions: reaction volume 100 µl, 10 µl reaction buffer T4 DNA ligase 10x (NEB), 3 µg DNA, 60 units Bsal-HFv2 (NEB), 2000 units T4 DNA ligase (NEB), 480 ng DNA adaptor molecule (1:20 molar excess), incubation time and temperature: 5 minutes at 37°C and 5 minutes at 16°C (60 cycles). Exonuclease clean-up reaction conditions: 15 units of *E. coli* exonuclease I (NEB) and 75 units of *E. coli* exonuclease III (NEB) are then added to remove remaining adaptor molecules and open DNA molecules. Incubation time and temperature: 30 minutes at 37°C and 20 minutes at 80°C.

### Example 2: purification of a linear DNA product comprising biotinylated nucleotides

1000 µl of the DNA product precursor having closed ends and comprising one biotinylated nucleotide within each of the hairpin adaptor molecules (SEQ ID NO:10) was loaded onto a streptavidin column. The column was washed with a buffer comprising 50mM Tris-HCI,pH7.5 and 50mM NaCl. Analysis of the wash buffer showed that none of the DNA product precursor flowed through, indicating binding of the closed linear DNA product precursor comprising biotinylated nucleotides to the streptavidin column. Three separate 1000µl volumes of distilled water at 80°C were applied by hand to the column to elute the closed linear DNA product and obtain the purified closed linear DNA product.

## Claims

1. A method for purification of a linear DNA product wherein the method comprises:
(a) contacting a linear double-stranded DNA molecule with a ligase and a first adaptor molecule, to form a linear DNA product precursor comprising a linear double-stranded region and a first adaptor molecule appended to a first end of the linear region, wherein the first adaptor molecule comprises a first binding moiety;
(b) contacting the linear DNA product precursor with a first substrate under conditions in which the first binding moiety binds to the first substrate;
(c) washing the first substrate to remove unbound linear double-stranded DNA molecules; and
(d) obtaining a purified linear DNA product.

2. The method of claim 1, wherein the method further comprises appending a second adaptor molecule to a second end of the linear double-stranded region.

3. The method of claim 2, wherein the second adaptor molecule comprises a second binding moiety.

4. The method of any one of claims 1 to 3, wherein the first and/or second adaptor molecule is a DNA adaptor molecule.

5. The method of any one of claims 2 to 4, further comprising contacting the linear DNA product with a second substrate under conditions in which the second binding moiety binds to the second substrate.

6. The method of any one of claims 1 to 5, wherein the first and/or second adaptor molecule has
(a) a double stranded region and an overhang;
(b) a double stranded region and a blunt end; and/or
(c) a double stranded region and a hairpin.

7. The method of any one of claims 1 to 6, wherein the linear double-stranded region comprises a first strand and a second strand, and the linear DNA product precursor does not comprise a binding moiety on the first strand, or does not comprise a binding moiety on the second strand

8. The method of claim any one of claims 1 to 7, wherein linear DNA product is single stranded and the step of obtaining the purified linear DNA product is performed under denaturing conditions.

9. The method of any one of claims 1 to 7, wherein the linear DNA product is single stranded and contacting the linear DNA product precursor with a first and/or second substrate is performed under denaturing conditions.

10. The method of any one of claims 1 to 9, wherein the first adaptor molecule and/or second adaptor molecule comprises a cleavable endonuclease target sequence between the linear double stranded region and the binding moiety.

11. The method of claim 10, wherein the step of obtaining the purified linear DNA product is performed by releasing the linear DNA product from the substrate by endonuclease digestion with a restriction endonuclease having a target sequence in the first and/or second adaptor molecule.

12. The method of any one of claims 1 to 10, wherein the step of obtaining the purified linear DNA product is performed under eluting conditions.

13. The method of any one of claims 1 to 12, wherein the linear DNA product is double stranded and comprises a cassette and at least two phosphorothioated nucleotides at internal positions in each strand, wherein the at least two phosphorothioated nucleotides at internal positions in each strand are selected from:
(a) a 5'-end nucleotide of the cassette;
(b) a 3'-end nucleotide of the cassette; and
(c) one or more nucleotides outside of the cassette.

14. The method of any one of claims 2 to 13, wherein the linear double-stranded region is closed at a first end by the first adaptor molecule and closed at a second end by the second adaptor molecule.

15. The method of any one of claims 2 to 13, wherein the first adaptor molecule comprises one or more nuclease-resistant nucleotides (i.e. protected nucleotides) and wherein the linear double-stranded region is closed at the second end by the second adaptor molecule.

16. The method of any one of claims 2 to 13, wherein the first and second adaptor molecules each comprise one or more nuclease-resistant nucleotides (i.e. protected nucleotides).

17. The method of any one of claims 1 to 16, wherein the first and/or second adaptor molecule comprises a biotinylated nucleotide, an aptamer, a homopolymeric sequence, an antibody or antibody binding fragment, a His Tag, or a GST tag.

18. Use of a purified linear DNA product in the production of a viral or non-viral delivery system, wherein the purified linear DNA product is purified by performing the method of any one of claims 1 to 17.
